# EUROPEAN PATENT SPECIFICATION

(11) **EP 1 226 131 B1**
(45) Date of publication and mention of the grant of the patent: **17.12.2003**
(21) Application number: 00992436.6
(22) Date of filing: 16.10.2000
(51) Int. Cl.: C07D 261/18, A61K 31/42, A61P 13/08

(54) **ISOXAZOLECARBOXAMIDE DERIVATIVES**
ISOXAZOLCARBOXAMID-DERIVATE ALS ANTAGONISTEN DES ALPHA1-ADRENERGISCHEN REZEPTORS
DERIVES D'ISOXAZOLECARBOXAMIDE

(30) Priority: 18.10.1999 IT MI992173
(43) Date of publication of application: 31.07.2002
(73) Proprietor: RECORDATI INDUSTRIA CHIMICA E FARMACEUTICA S.p.a., 20148 Milano (IT); RECORDATI S.A., 6830 Chiasso (CH)
(72) Inventor: LEONARDI, Amedeo, I-20154 Milano (IT); MOTTA, Gianni, I-20030 Barlassina (IT); RIVA, Carlo, I-21100 Varese (IT); POGGESI, Elena, I-20148 Milano (IT)
(74) Representative: McKelvey, Ian Edward
(86) International application number: EP0010144
(87) International publication number: WO01029015

(56) References cited:
- WO-A-95/04049
- WO-A-96/02246
- US-A- 5 403 842
- NAGARATHNAM D ET AL: "DESIGN AND SYNTHESIS OF NOVEL ALPHAA1 ADRENOCEPTOR-SELECTIVE DIHYDROPYRIDINE ANTAGONISTS FOR THE TREATMENT OF BENIGN PROSTATIC HYPERPLASIA" JOURNAL OF MEDICINAL CHEMISTRY,AMERICAN CHEMICAL SOCIETY. WASHINGTON,US, vol. 41, no. 26, 1998, pages 5320-5333, XP000941531 ISSN: 0022-2623

## Description

### SCOPE OF THE INVENTION

The invention relates to isoxazolecarboxamide derivatives, to pharmaceutical compositions containing them and to uses for such derivatives and compositions.

### BACKGROUND OF THE INVENTION

US 5403842 and its continuations (US 5474994 and US 5605896) claim heterobicyclic derivatives bearing substituted phenylpiperazines linked to the heterocyclic ring by a variety of spacer groups. Among said derivatives, Compound A (Example 11; Rec 15/2739) is of particular interest due its high activity and uroselectivity.

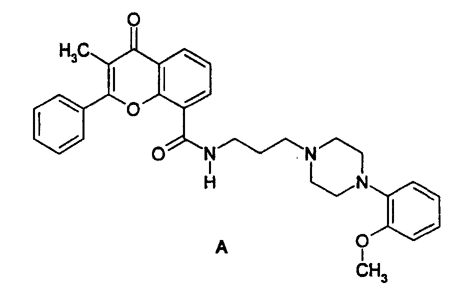

Compound A is endowed with good affinity for the α_{1A} adrenoceptor and is able to selectively inhibit contractility of the prostatic urethra in a dog model without substantial effects on blood pressure (Leonardi A. et al., *J. Pharmacol. Exp. Therap*. 281, 1272-1283 (1997).

*N*,ω-Aminoalkylamides of 5-methyl-3-phenyl-4-isoxazolecarboxylic acid are known compounds, but prior art molecules possess quite diverse molecular structures as compared to those claimed in this patent and a completely different mechanism of action. For example, EP 0573883 includes, as a representative compound, 3-(2-chloro-6-fluorophenyl)-N-[3-(2-chlorophenylamino)-propyl]-5-methylisoxazole-4-carboxamide and other similar derivatives and their claimed therapeutic application is the care of endoparasitoses. EP 0428434 claims 3-(2-chlorophenyl)-N-{2-(3,4-dichlorophenyl)-4-[4-(phenylmethyl)-1-piperidinyl]-butyl}-5-methylisoxazole-4-carboxamide as substance P antagonists. Neither of these patents claim arylpiperazinyl derivatives active at the α₁ adrenergic receptor.

The invention is directed to the structural class of N-(substituted phenyl)-N'-{ω-[3-(optionally substituted phenyl)-4-isoxazolecarbonylamino]-alkyl}-piperazines. The compounds of this class are endowed with enhanced selectivity toward the α₁ₐ adrenergic receptor, in particular with respect to the 5-HT_{1A} receptor, and improved *in vivo* uroselectivity even compared to compound A, with remarkable effects on relaxation of prostatic urethra and very low activity in lowering blood pressure. This activity profile suggests the safer use of the compounds of the invention in the therapy of obstructive syndromes of the lower urinary tract, including benign prostatic hyperplasia (BPH); of lower urinary tract symptoms (LUTS); and of neurogenic lower urinary tract dysfunction (NLUTD); without side-effects associated with hypotensive activity.

### SUMMARY OF THE INVENTION

The invention provides compounds of the general formula I: 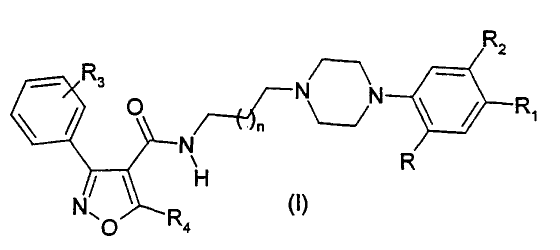 wherein:
R represents an alkyl, alkoxy, polyfluoroalkoxy, hydroxy or trifluoromethanesulphonyloxy group;
each of R₁ and R₂ independently represents a hydrogen or halogen atom or a polyfluoroalkoxy or alkoxy group;
R₃ represents one or more substituents consisting of a hydrogen or halogen atom or an alkyl, alkoxy, nitro, amino, acylamino, cyano, alkoxycarbonyl, carboxamido group;
R₄ represents a hydrogen atom or an alkyl or aralkyl group; and
n is 0, 1 or 2.

The invention also includes the N-oxides and pharmaceutically acceptable salts of these compounds.

The preferred alkyl groups which R₄ may represent are lower alkyl groups, preferably the methyl group. The preferred alkoxy groups which R, R₁, R₂ and R₃ may represent are lower alkoxy groups, preferably the methoxy group. Preferred polyfluoroalkoxy groups which R, R₁ and R₂ may represent are trifluoromethoxy or 2,2,2-trifluoroethoxy groups. The preferred value for n is 1.

The invention further provides pharmaceutical compositions comprising a compound of the general formula I or an N-oxide or pharmaceutically acceptable salt of such a compound, in admixture with a pharmaceutically acceptable diluent or carrier.

In another aspect, the invention is directed to methods for selectively preventing contractions (including noradrenaline-related contractions) of the urethra and lower urinary tract, without substantially affecting blood pressure, by administering one or more selected compounds of the general formula I to a mammal (including a human) in need of such treatment in an amount or amounts effective for the particular use.

In yet another aspect, the invention is directed to methods for blocking α₁ receptors, by delivering to the environment of said receptors, e.g. to the extracellular medium, (or by administering to a mammal possessing said receptors) an effective amount of a compound of the invention, in this way relieving diseases associated with overactivity of said receptors.

### DETAILED DESCRIPTION OF THE INVENTION

All patents, patent applications, and literature references cited in this application are incorporated by reference in their entirety.

The adrenergic antagonistic activity of the compounds of the invention renders them useful as agents acting on body tissues particularly rich in α₁ adrenergic receptors (such as prostate and urethra). Accordingly, anti-adrenergic compounds within the invention, established as such on the basis of their receptor binding profile, can be useful therapeutic agents for the treatment, for example, of micturition problems associated with obstructive disorders of the lower urinary tract, including but not limited to benign prostatic hyperplasia (BPH).

BPH is a progressive condition, which is characterised by a nodular enlargement of prostatic tissue resulting in obstruction of the urethra. This results in increased frequency of urination, nocturia, a poor urinary stream and hesitancy or delay in starting urine flow. Chronic consequences of BPH can include hypertrophy of bladder smooth muscle, a decompensated bladder and an increased incidence of urinary tract infection. The specific biochemical, histological and pharmacological properties of the prostate adenoma leading to bladder outlet obstruction are not yet known. However, the development of BPH is considered to be an inescapable phenomenon for the ageing male population. BPH is observed in approximately 70% of males over the age of 70. Currently, the worldwide stated method of choice for treating BPH is surgery. A medicinal alternative to surgery is clearly very desirable. The limitations of surgery for treating BPH include the morbidity rate of an operative procedure in elderly men, persistence or recurrence of obstructive and irritative symptoms, as well as the significant cost of surgery.

α-Adrenergic receptors (McGrath, et. al., *Med. Res. Rev*. 9, 407-533, (1989)) are specific neuroreceptor proteins located in the peripheral and central nervous systems on tissues and organs throughout the body. These receptors are important targets for controlling many physiological functions and thus represent important targets for drug development.

In fact, many α-adrenergic drugs have been developed over the past 40 years. Examples include clonidine, phenoxybenzamine and prazosin, terazosin, alfuzosin, doxazosin, tamsulosin (treatment of hypertension), naphazoline (nasal decongestant), and apraclonidine (treating glaucoma). α-Adrenergic drugs can be broken down into two distinct classes: agonists (clonidine and naphazoline are agonists), which mimic the receptor activation properties of the endogenous neurotransmitter noradrenaline, and antagonists (phenoxybenzamine and prazosin, terazosin, alfuzosin, doxazosin and tamsulosin are antagonists), which act to block the effects of noreadrenaline. Many of these drugs are effective but also produce unwanted side effects (for example, clonidine produces dry mouth and sedation in addition to its antihypertensive effects).

The above reported agonists are selective for the α₂ adrenergic receptor whereas most antagonists are selective for the α₁ adrenoceptor, with the exception of tamsulosin which shows a relevant affinity also for the 5-HT_{1A} receptor. Many of the cited α₁ antagonists are currently used for the therapy of BPH but, due to their poor uroselectivity, they are liable to cause side effects of cardiovascular type.

Recent pharmacological, biochemical and radioligand-binding studies evidenced three different α₁-receptor subtypes with a high affinity for prazosin, namely α_{1A}- (α₁ₐ-), α_{1B}-(α_{1b}-) and α_{1D}- (α_{1d}-), with lower case subscripts being used for recombinant receptors and upper case subscripts for receptors in native tissues (Hieble et al., *Pharmacol. Rev*. 47, 267-270, 1995). In functional studies α₁ receptors with a low affinity for prazosin have also been identified and termed α_{1L} receptors (Flavahan N. A. et al., *Trends Pharmacol. Sci*. 7, 347-349 (1986); Muramatsu et al., *Pharmacol. Comm*. 6, 23-28 (1995)).

Several studies have demonstrated the presence of these α₁-adrenergic-receptor subtypes in the lower-urinary-tract tissues (Andersson K. E., *4*^{*th*} *International Consultation in Benign Prostatic Hyperplasia (BPH)*", Paris, July 2-5, 1997, pages 601-609).

Several studies have shown that the human prostate receives innervation from both the sympathetic and parasympathetic nervous systems.

The adrenergic nerves are considered responsible for prostatic smooth-muscle tone by releasing noradrenaline, stimulating contraction-mediating α₁-adrenergic receptors. Approximately 50% of the total urethral pressure in BPH patients may be due to α₁-adrenoceptor-mediated muscle tone. Functional studies have indicated the occurrence of important adrenoceptor functions in prostatic adenomatous and capsular tissue. Clinical studies with the prototypical α₁-adrenoceptor-selective antagonist, prazosin, enforced the key role of α₁ adrenoceptors in the control of prostatic smooth-muscle tone. This was also confirmed in the laboratory by studies showing that, although both α₁ and α₂ adrenoceptors can be identified within the human prostate, contractile properties are mediated primarily by α₁ adrenoceptors. Many clinical investigations have confirmed that α₁-adrenoceptor blockade relieves lower-urinary-tract symptoms (LUTS), both of irritative and obstructive type, in patients with BPH.

Lower urinary tract dysfunction symptoms (LUTS) also develop in women as they age. As in men, LUTS in women includes both filling symptoms such as urgency, incontinence and nocturia, and voiding symptoms such as weak stream, hesitancy, intermittency, incomplete bladder emptying and abdominal straining. That both men and women experience a similar high prevalence of filling and voiding LUTS suggests that at least part of the underlying etiology may be identical. In a recent study, an α₁-antagonist was reported to reduce LUTS in women to a greater extent than an anticholinergic (Serels, S. and Stein, M., *Neurology and Urodynamics* 17: 31-36 (1998)). The authors concluded that there appeared to be a role for α₁-antagonists in treating LUTS in women. The possible mechanisms implicated to explain these results are: a) dysfunction of the bladder neck and urethra, causing functional outlet obstruction, analogous to BPH-induced outlet obstruction, with secondary detrusor overactivity; and b) increased α₁-adrenoreceptor activity in the detrusor, causing frequency and urgency. On these bases, α₁-antagonists are used in clinical practice to treat LUTS in women (Fitzpatrick, *International British J. Urol*. 85, Supp. 2: 1-5 (2000); Kakizaki, M. *et al., Brit. J. Urol. International* 85, Supp. 2: 25-30 (2000)). The results of Serels also indicate that the combined administration of α₁-antagonists and anticholinergics can have improved efficacy in treatment of LUTS, as suggested by Fitzpatrick, *International British J. Urol*. 85, Supp. 2: 1-5 (2000).

Another possible use of α₁-antagonists is the management of neurogenic lower urinary tract dysfunction (NLUTD), as can be caused by neurological disease or trauma. NLUTD may lead to debilitating symptoms and serious complications, including increased urinary frequency, incontinence, voiding difficulty, recurrent upper urinary tract infections and upper urinary tract deterioration. Management of NLUTD is indicated to preserve renal function and avoid urological complications. Administration of α₁-antagonists may benefit patients with NLUTD by facilitating urine storage by alleviating high detrusor pressure during bladder filling, which is evidenced by poor bladder compliance and detrusor hyperreflexia. In both animal models and patients with spinal cord injury resistant to anticholinergics, α₁-antagonists improved bladder compliance. (Kakizaki, M. *et al., Brit. J. Urol. International* 85, Supp. 2: 25-30 (2000); Sundin, T. *et al., Invest Urol*. 14: 322-328 (1977); McGuire *et al., Neurology and Urodynamics* 4: 139-142 (1985); Swrerzewski, S.J. *et al., J. Urol*. 151: 951-954 (1994)).

Two distinct α₁-adrenoceptor subtypes have been suggested to be present in the human prostate, one (α_{1H}) with high and one (α_{1L}) with low affinity for prazosin. All three high-affinity α₁-adrenoceptor subtypes found in molecular cloning studies have been identified in prostatic stromal tissue. The α₁ₐ subtype was found to be the dominant, representing about 60-85% of the α₁-adrenoceptor population. Recent findings suggest that there may be differences in subtype populations between normal and hyperplastic prostates, the ratios between the subtypes α₁ₐ:α_{1b}:α_{1d} being 85:1:14 in BPH tissue and 63:6:31 in non-BPH tissue.

The α_{1A} adrenoceptor was reported to mediate the contractile response of the human prostate *in vitro*. Ford A. P. D. W. et al., *Br. J. Pharmacol*. 114, 24 P (1995) found that the α_{1A} adrenoceptor may not mediate contractile responses to noradrenaline, and suggested as a candidate the α_{1L} adrenoceptor. Findings by Kenny B. A. et al., *Br. J. Pharmacol*. 118, 871-878 (1996), support the view that the α_{1L} adrenoceptor, which appears to share many of the characteristics of an α_{1A} adrenoceptor, mediates the contractile response of the human prostate.

In the female urethra, mRNA for the α₁ subtype was predominant and autoradiography confirmed the predominance of the α_{1A} adrenoceptor (Andersson, K.E., *Brit. J Urol. Intl.* 85, Supp. 2: 12-18 (2000)). The α_{1A} and α_{1D} subtypes are reported to be present in the human detrusor, with the latter subtype predominant (Malloy, B. *et al., J. Urol*. 160: 937-943 (1998)). Accordingly, the evidence that α₁ adrenoceptor antagonists are useful in treating lower urinary tract symptoms of both prostatic and non-prostatic origin in both males and females can be used to support the usefulness of the compounds of the present invention in treating such symptoms regardless of whether they are of obstructive origin or not and regardless of the sex of the patient.

On the other hand, it has also been suggested that the α_{1A} and α_{1L} adrenoceptors may represent distinct pharmacological forms of the same receptor.

The affinity of the compounds of the invention for each receptor can be assessed by receptor binding assays, for example as follows:
(1) α₁-adrenergic-receptor subtypes: using the specific ligand ³H-prazosin, according to Testa R. et al., *Pharmacol. Comm*. 6, 79-86 (1995);
(2) 5HT_{1A}-serotonergic receptors: using the specific ligand ³H-8-OH-DPAT according to Fargin A. et al., *Nature* 335, 358-360 (1988).

The α_{1L}-adrenergic receptor is not yet cloned and, therefore, the functional affinity of the compounds of the invention for this subtype can be assessed by using an isolated organ preparation as reported by Testa R. et al., *J Pharmacol. Exp. Ther*. 281, 1284-1293 (1997).

*In vitro* testing of the compounds of this invention on the above receptors is described in Examples 34 and 35 below.

The drugs having α₁-adrenergic antagonistic activity currently used for the symptomatic therapy of BPH are poorly subtype selective and subject to cause relevant side effects due to their hypotensive activity.

Thus there is a need for selective α₁-antagonists which do not subject the BPH patient to the side effects of said treatments, notably of the cardiovascular type.

The very high uroselectivity of the compounds of this invention has been tested in the dog model described in Example 36, where their efficacy in antagonising the contractions of prostatic urethra in the presence of very limited effects on blood pressure has been shown, in comparison to compound A and to another well-know α₁-antagonist, prazosin.

Accordingly, it is a primary object of the invention to provide a method of treating BPH which avoids any undue side effects due to acute hypotension.

It is another object of the invention to provide pharmaceutical compositions comprising isoxazole compounds which are selective α₁-adrenoceptor antagonists, which compositions are effective for the treatment of BPH.

It is another object of the invention to provide a method of treating BPH using isoxazole compounds which are selective α₁-adrenoceptor antagonists.

It is another object of the invention to provide a method for the treatment of neurogenic lower urinary tract dysfunction in patients and a method for the treatment of lower urinary tract symptoms in female patients, optionally further comprising the inclusion of an anticholinergic compound which may be selected from the group consisting of tolterodine, oxybutinin, darifenacin, alvameline and temiverine.

Another aspect of the invention is the use of new compounds for lowering intraocular pressure, inhibiting cholesterol synthesis, reducing sympathetically-mediated pain and the treatment of cardiac arrhythmia and erectile dysfunction.

Other features and advantages of the invention will be apparent to those skilled in the art from the following detailed description and claims.

### SYNTHESIS OF THE COMPOUNDS OF THE INVENTION

The compounds according to the invention may generally be prepared as follows:

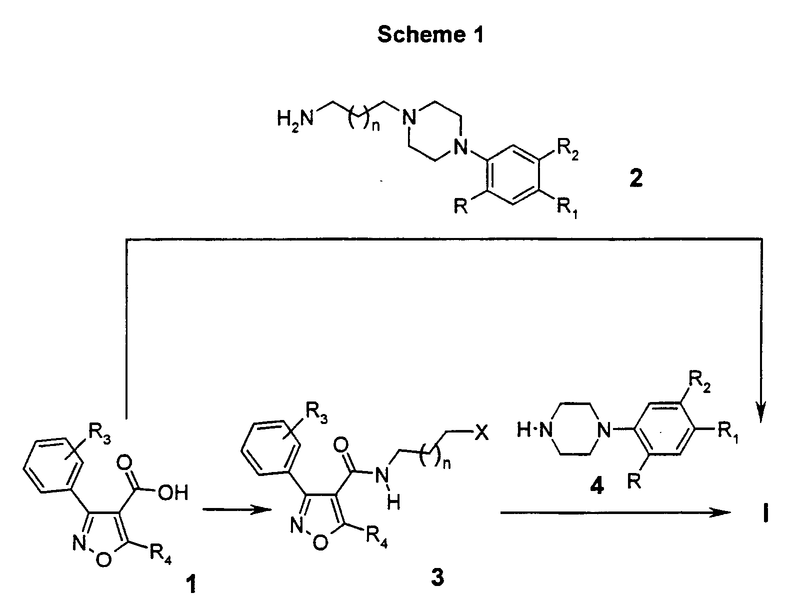

Direct condensation of compounds **1** with the ω-aminoalkyl derivatives **2** (Scheme 1) leads to the compounds of the invention. The condensation can be carried out in presence of a condensing agent (e.g. dicyclohexylcarbodiimide or diethyl cyanophosphonate) optionally in the presence of a promoting agent (e.g. N-hydroxysuccinimide, 4-dimethylaminopyridine or *N*,*N*'-carbonyldiimidazole) in an aprotic or chlorinated solvent (e.g. dimethylformamide or chloroform) at -10/140°C (Albertson N. F., *Org. React*. 12, 205-218 (1962); Doherty A. M. et al., *J. Med. Chem*. 35, 2-14 (1992); Ishihara Y. et al., *Chem. Pharm. Bull*. 39, 3236-3243 (1991)). In some cases the activated intermediate esters or amides (such as O-(N-succinimidyl)esters or acyl imidazolides) can be isolated and further reacted with **2** to be transformed into the corresponding amides (**I**) in an aprotic or chlorinated solvent at 10/100°C. This kind of condensation is well illustrated in the examples. Another activated intermediate which can be used is the mixed anhydride of **1**, obtainable by reacting **1** with an alkyl chloroformate in presence of a tertiary amine (e.g. triethylamine or *N*-methylmorpholine), then reacted with **2** at 0-80°C; optionally a promoting agent (e.g. 1-hydroxypiperidine) may be added before the amine addition (Albertson N. F., *Org. React*. 12, 157 (1962)).

Alternatively, the condensation can be carried out without a solvent at 150-220°C (Mitchell J. A. et al., *J. Am. Chem. Soc*. 53, 1879 (1931)) or in high-boiling ethereal solvents (e.g. diglyme).

The condensation can be also performed through preparation and optional isolation of reactive derivatives of **1,** such as acyl halides. The preparation and use of these last derivatives are well documented in the literature and known to people skilled in the art.

Also less reactive derivatives of **1** can be used, such as alkyl esters, which, in turn, can be converted into **I** in the presence of a condensing agent (e.g. trimethylaluminum) in an aprotic and/or a chlorinated solvent (e.g. hexane, dichloromethane) at -10/80°C, or without solvents at 80-180°C, (Weinreb S. M. et al., *Tetrahedron Lett.* 4171 (1977); Lipton M. F. et al., *Org. Synth*. 59, 49 (1979)).

By the same methods of condensation reported above and using H₂NCH₂(CH₂)ₙCH₂X (with X = halogen or OH) as a reagent, **1** can be transformed into **3.** In the case of X = OH, the alcoholic group is then converted into a suitable leaving group by methods well known to those skilled in the art. Compounds **3** (with X = leaving group such as halogen or aryl/alkylsulphonyloxy group) can be subsequently reacted with an appropriate phenylpiperazine **4.** The nucleophilic substitution is carried out preferably, but not necessarily at a temperature within the range of 20-200°C in a polar solvent such as dimethylformamide, acetonitrile or methanol, or without any solvent, usually in the presence of a base such as potassium carbonate. See also Gibson's chapter in Patai: "*The Chemistry of the Amino Group*", p. 45, Wiley Int. Sci., N.Y. (1968).

The preparation of compounds **2** is disclosed in the literature and is well known to those skilled in the art, and includes nucleophilic substitution of a phenylpiperazine **4** on a *N*-(ω-haloalkyl)phthalimide or a proper ω-haloalkylnitrile or amide by the method illustrated above for the condensation of compounds **3** and **4** or by addition of a α,β-unsaturated alkylnitrile or amide in a suitable solvent (e.g. acetonitrile, *N,N-*dimethylformamide, a chlorinated solvent or other aprotic polar solvent) at a temperature between 0°C and the reflux temperature of the solvent. The following standard phthalimido-group deprotection and the reduction of the amido or cyano group provide compounds **2**.

The compounds **I** where R is a trifluoromethanesulphonyloxy group can be synthesised starting from compounds **I** where R is a hydroxy group by known procedures that include the use of trifluoromethanesulphonic anhydride or *N*-phenyltrifluoromethanesulphonimide in aprotic solvents such as, for example, 1,2-dichloroethane or other chlorinated solvents, toluene at a temperature in the range between -20°C and the reflux temperature of the solvent (Hendrickson J. B. et al., *Tetrahedron Letters,* 4607-4610 (1973)).

The N-oxides of compounds **I** may be synthesised by simple oxidation procedures known to those skilled in the art. The oxidation procedure described by Brougham P., Synthesis, 1015-1017 (1987), allows differentiation of the two nitrogen atoms of the piperazine ring, permitting both the N-oxides and the N, N'-dioxide to be obtained.

The preparation of the phenylpiperazines **4**, not yet known in the literature, is very well documented in the experimental part and uses synthetic procedures very well known to those skilled in the art, which comprise the synthesis of the proper aniline through standard reactions and the subsequent cyclization with *bis*-(2-chloroethylamine) to afford the piperazine following the method of Prelog V. et al., *Collect. Czech. Chem. Comm*. 5, 497-502 (1933)) or its variations (Elworthy T. R., *J. Med. Chem*. 40, 2674-2687 (1997)).

### DETAILED SYNTHESIS

The following examples illustrate the invention, without limiting it.

### Example 1

### N-{3-[4-(5-Chloro-2-methoxyphenyl)-1-piperazinyl]-propyl}-5-methyl-3-phenylisoxazole-4-carboxamide

### a) 1-(5-Chloro-2-methoxyphenyl)-4-[3-(N-phthalimido)-propyl]-piperazine (Compound 1A)

A mixture of 28.64 g of 1-(5-chloro-2-methoxyphenyl)-piperazine, 44.6 g of anhydrous potassium carbonate and 33.65 g of N-(3-bromopropyl)-phthalimide in 250 ml of acetonitrile was stirred at reflux for 8 hours. After cooling to room temperature, 800 ml of water was added under stirring and the resulting suspension was filtered by suction yielding a yellowish solid, which was washed with 300 ml of water and crystallised from methanol affording 46.5 g (91%) of the title compound, melting at 131-133°C.
^{**1**}**H-NMR (200MHz, CDCl**_{**3**}**, δ):** 7.78-7.82, m, 2H, phthalimide H3, H6; 7.64-7.78, m, 2H, phthalimide H4, H5; 6.92, dd, 1H, methoxyphenyl H4; 6.65-6.78, m, 2H, methoxyphenyl H3, H6; 3.81, s, 3H, CH₃O; 3.71-3.89, m, 2H, CH₂N(CO)₂; 2.78-3.00, m, 4H, 2 piperazine CH₂s; 2.40-2.65, m, 6H, 2 piperazine CH₂s, CH₂CH₂CH₂N(CO)₂; 1.80-2.03, m, 2H, CH₂CH₂CH₂.

### b) 1-(3-Aminopropyl)-4-(5-chloro-2-methoxyphenyl)piperazine trihydrochloride · 2.15 H₂O (Compound 1B)

A solution of 20.7 g of Compound 1A and 8.6 ml of 85% hydrazine hydrate in 300 ml of 95% ethanol was stirred at reflux for 3.5 hours. Afterwards, the reaction mixture was cooled to room temperature, diluted with 400 ml of water, acidified with 37% hydrochloric acid (pH = 1) and stirred for 0.5 hours. The precipitated solid was collected by filtration and washed with 1N hydrochloric acid followed by water. The filtrate was concentrated by evaporation *in vacuo*, filtered, made basic by the addition of 35% sodium hydroxide at 0-5°C and extracted with diethyl ether. The organic layer was washed with brine, dried on sodium sulphate and evaporated to dryness *in vacuo* affording 13.6 g (96%) of the title compound as a base. Acidification of a solution of the base in chloroform with more than three equivalents of 3N ethanolic hydrogen chloride, followed by evaporation to dryness *in vacuo* and crystallisation of the residue from ethanol/diethyl ether 10:3 yielded the title compound, melting at 200-202°C.
^{**1**}**H-NMR (200MHz, CDCl**_{**3**}**, δ):** 11.20-11.50, br, 1H, NH⁺; 8.10-8.40, br, 3H, NH₃⁺; 6.85-7.10, m, 3H, phenyl H3, H4, H6; 5.10, br, 5.3H, NH⁺, 2.15 H₂O; 3.79, s, 3H, CH₃O; 3.35-3.65, m, 4H, 2 piperazine CH₂s; 3.03-3.35, m, 6H, 2 piperazine CH₂s, CH₂CH₂CH₂NH₃⁺; 2.80-3.03, m, 2H, CH₂CH₂CH₂NH₃⁺; 1.95-2.22, m, 2H, CH₂CH₂CH₂NH₃⁺.

### c) N-(3-[4-(5-Chloro-2-methoxyphenyl)-1-piperazinyl]-propyl}-5-methyl-3-phenylisoxazole-4-carboxamide

1.08 g of 93% diethyl cyanophosphonate and 0.92 ml of triethylamine were added to a mixture of 1.22 g of 3-phenyl-5-methylisoxazole-4-carboxylic acid, 1.87 g of Compound 1B as its base and 30 ml of anhydrous dimethylformamide stirred at 0-5°C. The temperature was allowed to rise to 20-25°C and, after 3.5 hours' stirring, the mixture was poured into 300 ml of water and extracted with ethyl acetate. The combined organic layers were washed with 5% aqueous sodium carbonate and water. After drying on sodium sulphate, the solvent was removed *in vacuo.* The crude was crystallised from ethanol to yield 2.11 g (75%) of the title compound, melting at 139-142°C.
^{**1**}**H-NMR (200MHz, CDCl**_{**3**}**, δ):** 7.60-7.70, m, 2H, phenyl H2, H6; 7.45-7.55, m, 3H, phenyl H3, H4, H5; 6.95, dd, 1H, methoxyphenyl H4; 6.85, d, 1H, methoxyphenyl H6; 6.75, d, 1H, methoxyphenyl H3; 6.25, t, 1H, NH; 3.82, s, 3H, OCH₃; 3.40, q, 2H, NHCH₂; 2.80-2.95, m, 4H, 2 piperazine CH₂s; 2.69, s, 3H, CH₃; 2.40-2.55, m, 4H, 2 piperazine CH₂s; 2.30, t, 2H, CONHCH₂CH₂CH₂N; 1.55-1.70, m, 2H, CH₂CH₂CH₂.

### Example 2

### N-{3-[4-(5-Chloro-2-hydroxyphenyl)-1-piperazinyl]-propyl}-5-methyl-3-phenylisoxazole-4-carboxamide

### a) 1-(5-Chloro-2-hydroxyphenyl)piperazine dihydrobromide (Compound 2A)

A suspension of 5.5 g of 1-(5-chloro-2-methoxyphenyl)-piperazine in 40 ml of 62% hydrobromic acid was stirred at reflux for 30 hours. After cooling to room temperature, the mixture was filtered by suction and the solid was washed on the funnel with acetone affording 6.02 g of the title compound. M.p. >270°C (ethanol).
^{**1**}**H-NMR (200MHz, CDCl**_{**3**}**, δ):** 9.35-9.80, br, 2H, NH₂⁺; 8.60-9.05, br, 2H, NH⁺, OH; 6.70-6.97, m, 3H, aromatic CHs; 3.00-3.38, m, 8H, piperazine CH₂s.

### b) N-(3-Chloropropyl)-3-phenyl-5-methylisoxazole-4-carboxamide (Compound 2B)

The title compound was synthesised following the procedure described for the compound of Example 1c, but substituting 3-chloropropylamine hydrochloride for Compound 1B and doubling the amount of triethylamine. Pouring the reaction mixture into iced water and filtering the precipitated solid, which was washed on the funnel with a 2:1 water : dimethylformamide mixture followed by water, afforded, after drying, the pure title compound. M.p. 122-124°C.
^{**1**}**H-NMR (200MHz, CDCl**_{**3**}**, δ):** 7.45-7.60, m, 5H, phenyl CHs; 5.50, br, 1H, NH; 3.30-3.45, m, 4H, CH₂CH₂CH₂; 2.70, s, 3H, CH₃; 1.80-1.90, m, 2H, CH₂CH₂CH₂.

### c) N-{3-[4-(5-Chloro-2-hydroxyphenyl)-1-piperazinyl]-propyl}-5-methyl-3-phenylisoxazole-4-carboxamide

A mixture of 0.28 g of Compound 2B, 0.21 g of Compound 2A and 0.14 g of potassium carbonate was heated at 160°C for 20 minutes. After cooling to room temperature, the mixture was taken up with 8 ml of chloroform, and inorganic matter was filtered off. The filtrate was evaporated to dryness and purified by flash chromatography (chloroform : methanol 97.5:2.5) to yield 0.32 g (71%) of the title compound. M.p. 92-98°C.
^{**1**}**H-NMR (200MHz, CDCl**_{**3**}**, δ):** 7.60-7.75, m, 2H, phenyl H2, H6; 7.45-7.60, m, 3H, phenyl H3, H4, H5; 7.00-7.10, m, 2H, hydroxyphenyl H4, H6; 6.85, d, 1H, hydroxyphenyl H3; 6.70, br, 1H, OH; 6.02, t, 1H, NH; 3.35, q, 2H, NHCH₂; 2.62-2.75, m, 7H, 2 piperazine CH₂s and CH₃; 2.38-2.50, m, 4H, 2 piperazine CH₂s; 2.30, t, 2H, CONHCH₂CH₂CH₂N; 1.55-1.70, m, 2H, CH₂CH₂CH₂

### Example 3

### 5-Methyl-3-phenyl-N-{3-[4-[2-(2,2,2-trifluoroethoxy)-phenyl]-1-piperazinyl]-propyl}isoxazole-4-carboxamide

This compound was prepared as described in Example 2c, substituting 1-[2-(2,2,2-trifluoroethoxy)-phenyl]-piperazine (prepared as described in EP 748800) for Compound 2A. The crude was purified by flash chromatography (ethyl acetate : methanol 95:5) to afford the title compound (47%). M.p. 124-125°C.
^{**1**}**H-NMR (200MHz, CDCl**_{**3**}**, δ):** 7.60-7.70, m, 2H, phenyl H2, H6; 7.40-7.55, m, 3H, phenyl H3, H4, H5; 6.88-7.05, m, 4H, trifluoroethoxyphenyl CHs; 6.35, t, 1H, NH; 4.35, q, 2H, OCH₂CF₃; 3.40, q, 2H, NHCH₂; 2.80-2.95, m, 4H, 2 piperazine CH₂s; 2.65, s, 3H, CH₃; 2.30-2.45, m, 4H, 2 piperazine CH₂s; 2.30, t, 2H, CONHCH₂CH₂CH₂N; 1.55-1.70, m, 2H, CH₂CH₂CH₂.

### Example 4

### N-{3-[4-[2-Methoxy-5-(2,2,2-trifluoroethoxy)phenyl]-1-piperazinyl]-propyl}-5-methyl-3-phenylisoxazole-4-carboxamide

### a) 1-(t-Butoxycarbonyl)-4-(5-hydroxy-2-methoxyphenyl)-piperazine (Compound 4A)

A solution of 8 g of 1-(5-hydroxy-2-methoxyphenyl)piperazine dihydrobromide (prepared as described in US 5605896) and 3.17 g of anhydrous potassium carbonate in 30 ml of water was evaporated to dryness *in vacuo.* 100 ml of anhydrous tetrahydrofuran and 5.18 g of di-*t*-butyl dicarbonate were added to the residue and the mixture was stirred at room temperature for 2 hours, then 100 ml of anhydrous tetrahydrofuran was added. The suspension was filtered and the solvent was removed *in vacuo*. The residue was dissolved in 200 ml of chloroform, the solution was washed with 3 × 50 ml of 5% sodium bicarbonate, 2 × 50 ml of water and dried over sodium sulphate. The solvent was removed at reduced pressure and the residue was purified by flash chromatography (petroleum ether : ethyl acetate 75:25) to give 1.91 g (28.7%) of Compound 4A and 1.58 g (35.7%) of 1-(*t*-butoxycarbonyl)-4-[5-(t-butoxycarbonyloxy)-2-methoxyphenyl]-piperazine. A solution of this by-product, 40 ml of methanol and 6 ml of 1N sodium hydroxide was maintained overnight at room temperature. The mixture was neutralised with acetic acid; the solvent was removed at reduced pressure and the residue dissolved in 40 ml of chloroform. After washing with 3 × 10 ml of water, the organic layer was dried over sodium sulphate and the solvent evaporated off *in vacuo* to recover an additional 1.15 g (17.2%) of Compound 4A (total 45.9%).
^{**1**}**H-NMR (200MHz, CDCl**_{**3**}**, δ):** 6.70, d, 1H, phenyl H3; 6.45-6.53, m, 2H, phenyl H4, H6; 5.77, br, 1H, OH; 3.78, s, 3H, CH₃O; 3.48-3.68, m, 4H, 2 piperazine CH₂s; 2.82-3.05, m, 4H, 2 piperazine CH₂s; 1.48, s, 9H, (CH₃)₃C.

### b) 1-(t-Butoxycarbonyl)-4-[2-methoxy-5-(2,2,2-trifluoroethoxy)-phenyl]piperazine (Compound 4B)

A stirred mixture of 2.83 g of Compound 4A, 6.05 g of cesium carbonate and 2.95 g of 2,2,2-trifluoroethyl *p*-toluenesulphonate in 60 ml of acetonitrile was refluxed for 16 hours. The solvent was evaporated at reduced pressure. 90 ml of brine was added to the residue and the mixture was extracted with 3 × 40 ml of ethyl acetate. The organic layer was washed with 3 × 20 ml of water and 20 ml of brine, and dried over sodium sulphate. The solvent was removed at reduced pressure and the residue purified by flash chromatography (petroleum ether/ethyl acetate gradient 95:5 to 80:20). The solvents were removed *in vacuo* to give 1.86 g (52%) of Compound 4B as a white solid. M.p. (98) 102-105°C.
^{**1**}**H-NMR (200MHz, CDCl**_{**3**}**, δ):** 6.77, d, 1H, phenyl H3; 6.45-6.63, m, 2H, phenyl H4, H6; 4.28, q, 2H, CF₃CH₂O; 3.84, s, 3H, CH₃O; 3.53-3.68, m, 4H, 2 piperazine CH₂s; 2.90-3.06, m, 4H, 2 piperazine CH₂s; 1.48, s, 9H, (CH₃)₃C.

### c) 1-[2-Methoxy-5-(2,2,2-trifluoroethoxy)-phenyl]piperazine · 1.9 HCl (Compound 4C)

A solution of 2.42 ml of trifluoroacetic acid in 30 ml of anhydrous dichloromethane was added dropwise at 3-5°C to a stirred solution of 1.17 g of Compound 4B in 40 ml of anhydrous dichloromethane. The mixture was maintained overnight at room temperature, washed with 2 × 30 ml of 2N sodium hydroxide and extracted with 3 × 15 ml of 2N hydrochloric acid. The aqueous acid layer was washed with 2 × 20 ml of diethyl ether, alkalinised with 37% sodium hydroxide at 5-10°C and extracted with 3 × 30 ml of diethyl ether. The organic layer was dried over sodium sulphate and the solvent was removed *in vacuo* to give 0.78 g (89%) of compound 4C base as a thick oil. A solution of this base in diethyl ether was treated with charcoal, filtered and acidified by addition of 3.6N hydrogen chloride in diethyl ether to give the hydrochloride salt, recovered by filtration and crystallised from acetonitrile and ethanol (7.5:1) to yield the analytical sample. M.p. (188) 202-208°C (dec.).
^{**1**}**H-NMR (200MHz, CDCl**_{**3**}**, δ):** 9.18, br, 1.9H, NH₂⁺: 6.90, d, 1H, phenyl H3; 6.67, dd, 1H, phenyl H4; 6.59, d, 1H, phenyl H6; 6.11, br, 1H, NH⁺; 4.66, q, 2H, CF₃CH₂O; 3.74, s, 3H, CH₃O; 3.18, br, 8H, piperazine CH₂s.

### d) N-{3-[4-[2-Methoxy-5-(2,2,2-trifluoroethoxy)-phenyl]-1-piperazinyl]-propyl}-5-methyl-3-phenylisoxazole-4-carboxamide

This compound was prepared as described in Example 2c, but substituting Compound 4C for Compound 2A. After cooling to room temperature, the crude was purified by flash chromatography (dichloromethane : 2N ammonia in methanol 97.5:2.5) to afford the title compound (54%). M.p. 125°C.
^{**1**}**H-NMR (200MHz, CDCl**_{**3**}**, δ):** 7.65-7.75, m, 2H, phenyl H2, H6; 7.45-7.55, m, 3H, phenyl H3, H4, H5; 6.75, d, 1H, trifluoroethoxyphenyl H3; 6.45-6.55, m, 2H, trifluoroethoxyphenyl H4, H6; 6.20, t, 1H, NH; 4.30, q, 2H, OCH₂CF₃; 3.80, s, 3H, OCH₃; 3.35, q, 2H, NHCH₂; 2.80-2.95, m, 4H, 2 piperazine CH₂s; 2.65, s, 3H, CH₃; 2.35-2.48, m, 4H, 2 piperazine CH₂s; 2.27, t, 2H, CONHCH₂CH₂CH₂N; 1.52-1.68, m, 2H, CH₂CH₂CH₂.

### Example 5

### N-{3-[4-[5-Fluoro-2-(2,2,2-trifluoroethoxy)-phenyl]-1-piperazinyl]-propyl}-5-methyl-3-phenylisoxazole-4-carboxamide

### a) 5-Fluoro-2-(2,2,2-trifluoroethoxy)-nitrobenzene (Compound 5A)

A stirred mixture of 3.14 g of 4-fluoro-2-nitrophenol, 13 g of caesium carbonate and 20 ml of anhydrous dimethylformamide was heated at 100°C for 4 hours. After this period, 6.65 g of 2,2,2-trifluoroethyl *p*-toluenesulphonate was added and the mixture was stirred at the same temperature for 40 hours. Afterwards, the solvent was removed under reduced pressure at 35°C and 50 ml of water was added to the residue. The mixture was acidified with 37% hydrochloric acid and extracted with 3 × 40 ml of ethyl acetate. The organic layer was washed with 20 ml of brine, dried over sodium sulphate and evaporated to dryness *in vacuo*. The residue was purified by flash chromatography (petroleum ether : ethyl acetate 100:7) to afford 1.53 g (32%) of Compound 5A as an oil.
^{**1**}**H-NMR (200MHz, CDCl**_{**3**}**, δ):** 7.65, dd, 1H, H6; 7.32, ddd, 1H, H4; 7.16, dd, 1H, H3; 4.42, q, 2H, CH₂.

### b) 5-Fluoro-2-(2,2,2-trifluoroethoxy)-aniline (Compound 5B)

A mixture of 0.66 g of Compound 5A and 0.07 g of Raney-Nickel in 20 ml of ethyl acetate was stirred for 14 hours at 20-25°C. The organic layer was separated and the mixture extracted with 2 × 40 ml of ethyl acetate. The combined organic layers were washed with 20 ml of brine, dried over sodium sulphate and evaporated to dryness *in vacuo* to afford 0.52 g (90.6%) of Compound 5B as an orange oil.
^{**1**}**H-NMR (200MHz, CDCl**_{**3**}**, δ):** 6.70, dd, 1H, H6; 6.28-6.50, m, 2H, H3 and H4; 4.32, q, 2H, CH₂; 3.92, br, 2H, NH₂.

### c) 1-[5-Fluoro-2-(2,2,2-trifluoroethoxy)-phenyl]piperazine (Compound 5C)

A stirred mixture of 0.52 g of Compound 5B, 0.45 g of *bis*-(2-chloroethyl)amine hydrochloride, 0.5 g of potassium iodide, 0.34 g of anhydrous potassium carbonate and 20 ml of *n*-butanol was refluxed for 32 hours under nitrogen. The solvent was removed under reduced pressure. The residue was treated with 10 ml of water and 10 ml of 20% aqueous sodium carbonate and extracted with 2 × 30 ml of ethyl acetate. The organic layer was washed with brine, dried over sodium sulphate and evaporated to dryness *in vacuo*. The residue was purified by flash chromatography (chloroform : 2N ammonia in methanol gradient from 100:3 to 100:5) to afford 0.1 g (14 %) of Compound 5A as an oil.
^{**1**}**H-NMR (200MHz, CDCl**_{**3**}**, δ):** 6.80-6.93, m, 1H, H3; 6.55-6.71, m, 2H, H6, H4; 4.36, q, 2H, OCH₂CF₃; 3.05, br, 8H, piperazine CH₂s; 2.38, s, 1H, NH.

### d) N-{3-[4-[5-Fluoro-2-(2,2,2-trifluoroethoxy)-phenyl]-1-piperazinyl]-propyl}-5-methyl-3-phenylisoxazole-4-carboxamide

This compound was prepared as described in Example 2c, substituting Compound 5C for Compound 2A. After cooling to room temperature, the crude was purified by flash chromatography (dichloromethane : 2N ammonia in methanol 97.5:2.5) to afford the title compound (59 %). M.p. 123-125°C.
^{**1**}**H-NMR (200MHz, CDCl**_{**3**}**, δ):** 7.60-7.70, m, 2H, phenyl H2, H6; 7.45-7.55, m, 3H, phenyl H3, H4, H5; 6.80-6.90, m, 1H, trifluoroethoxyphenyl H3; 6.55-6.70, m, 2H, trifluoroethoxyphenyl H4, H6; 6.20, t, 1H, NH; 4.30, q, 2H, OCH₂CF₃; 3.35, q, 2H, NHCH₂; 2.80-2.95, m, 4H, 2 piperazine CH₂s; 2.65, s, 3H, CH₃; 2.35-2.45, m, 4H, 2 piperazine CH₂s; 2.25, t, 2H, CONHCH₂CH₂CH₂N; 1.50-1.65, m, 2H, CH₂CH₂CH₂.

### Example 6

### N-{3-[4-[4-Fluoro-2-(2,2,2-trifluoroethoxy)-phenyl]-1-piperazinyl]-propyl}-5-methyl-3-phenylisoxazole-4-carboxamide

This compound was prepared as described in Example 2, substituting 1-[4-fluoro-2-(2,2,2-trifluoroethoxy)-phenyl]piperazine (prepared as described in EP 748800) for Compound 2A. After cooling to room temperature, the crude was purified by flash chromatography (dichloromethane : 2N ammonia in methanol 98:2) to afford the title compound (64%). M.p. 112-135°C°.
^{**1**}**H-NMR (200MHz, CDCl**_{**3**}**, δ):** 7.60-7.70, m, 2H, phenyl H2, H6; 7.45-7.55, m, 3H, phenyl H3, H4, H5; 6.60-6.85, m, 3H, trifluoroethoxyphenyl H3, H5, H6; 6.28, t, 1H, NH; 4.20-4.40, m, 2H, OCH₂CF₃; 3.35, q, 2H, NHCH₂; 2.75-2.90, m, 4H, 2 piperazine CH₂s; 2.65, s, 3H, CH₃; 2.35-2.45, m, 4H, 2 piperazine CH₂s; 2.25, t, 2H, CONHCH₂CH₂CH₂N; 1.50-1.65, m, 2H, CH₂CH₂CH₂.

### Example 7

### N-{3-[4-[(5-Chloro-2-trifluoromethanesulphonyloxy)-phenyl]-1-piperazinyl]-propyl}-5-methyl-3-phenylisoxazole-4-carboxamide

0.07 ml of triethylamine was added at room temperature to a stirred mixture of 0.22 g of the Compound of Example 2, 0.18 g of N-phenyltriflimide and 5 ml of dichloromethane. The mixture was stirred for 10 days; during this period 2 additional amounts of N-phenyltriflimide (0.18 and 0.09 g) and of triethylamine (0.07 and 0.04 ml) were added. The reaction mixture was diluted with 5 ml of dichloromethane, washed with 10% aqueous sodium carbonate, water, dried over sodium sulphate and evaporated to dryness *in vacuo*. The crude was purified by flash chromatography (ethyl acetate : methanol 95:5) to afford 0.17 g (58%) of the pure title compound. M.p. 49-53°C.
^{**1**}**H-NMR (200MHz, CDCl**_{**3**}**, δ):** 7.65-7.75, m, 2H, phenyl H2, H6; 7.50-7.60, m, 3H, phenyl H3, H4, H5; 7.02-7.10, m, 3H, chlorophenyl H3, H4, H6; 6.20, t, 1H, NH; 3.35, q, 2H, NHCH₂; 2.75-2.85, m, 4H, 2 piperazine CH₂s; 2.70, s, 3H, CH₃; 2.40-2.50, m, 4H, 2 piperazine CH₂s; 2.32, t, 2H, CONHCH₂CH₂CH₂N; 1.55-1.70, m, 2H, CH₂CH₂CH₂.

### Example 8

### 3-(4-Fluorophenyl)-N-{3-[4-[4-fluoro-2-(2,2,2-trifluoroethoxy)-phenyl]-1-piperazinyl]-propyl}-isoxazole-4-carboxamide

### a) 1-[4-Fluoro-2-(2,2,2-trifluoroethoxy)-phenyl]-4-[3-(N-phthalimido)-propyl]-piperazine (Compound 8A)

The title compound was synthesized following the procedure described for Compound 1A of Example 1 but substituting 1-[4-fluoro-2-(2,2,2-trifluoroethoxy)-phenyl]-piperazine for 1-(5-chloro-2-methoxyphenyl)-piperazine. The reaction mixture was extracted with diethyl ether and the organic solution was dried over sodium sulphate and filtered on silica gel, washing the panel with diethyl ether. Evaporation to dryness in vacuo afforded the title product (66%), M.p. (104) 108-110°C.
^{**1**}**H-NMR (200MHz, CDCl**_{**3**}**, δ):** 7.60-7.95, m, 4H, phthalimido CHs; 6.52-6.90, m, 3H, fluorophenyl CHs; 4.35, q, 2H, OCH₂CF₃; 3.80, t, 2H, (CO)₂NCH₂; 2.70-3.20, m, 4H, 2 piperazine CH₂s; 2.30-2.70, m, 6H, (CO)₂NCH₂CH₂CH₂N, 2 piperazine CH₂s; 1.75-2.10, m, 2H, (CO)₂NCH₂CH₂.

### b) 1-(3-Aminopropyl)-4-[4-fluoro-2-(2,2,2-trifluoroethoxy)-phenyl]-piperazine (Compound 8B)

The title compound was synthesized following the procedure described for Compound 1B of Example 1 but substituting Compound 8A for Compound 1A. Extraction of alkalinized filtrate with dichloromethane afforded the title compound (84.8%) as an oil.
^{**1**}**H-NMR (200MHz, CDCl**_{**3**}**, δ):** 6.82-6.98, m, 1H, H5; 6.58-6.82, m, 2H, H3 and H6; 4.38, q, 2H, OCH₂CF₃; 2.92-3.12, m, 4H, 2 piperazine CH₂s; 2.80-2.92, m, 2H, H₂NCH₂CH₂CH₂N; 2.40-2.75, m, 8H, H₂NCH₂CH₂CH₂N, and 2 piperazine CH₂s; 1.62-1.82, m, 2H, H₂NCH₂CH₂CH₂N.

### c) Methyl 3-(4-fluorophenyl)-isozazole-4-carboxylate (Compound 8C)

To a solution of 1.05 g of 4-fluorobenzaldoxime (*Beil*. 7, I 132s, II 177d, III 863c) in 15 ml of chloroform were added 1.35 g of N-bromosuccinimide (NBS) and 0.04 ml of pyridine; the resulting mixture was stirred at room temperature for 4 h under nitrogen atmosphere. After cooling at 0°C, 1.75 g of methyl β-pyrrolidinoacrylate (prepared as described in US 4144047) and 1.26 ml of triethylamine were added and the suspension was stirred at room temperature for 18 h. The reaction mixture was diluted with 20 ml of 2N hydrochloric acid, 20 ml of water; the organic layer was separated, washed again with 2 N hydrochloric acid and water, dried (sodium sulphate), and evaporated to dryness *in vacuo*. The crude was purified by flash chromatography (toluene:dichloromethane 1:9) to afford the title compound (47%) as an oil.
^{**1**}**H-NMR (200MHz, CDCl**_{**3**}**, δ):** 9.02, s, 1H, isoxazole H5; 7.71-7.91, m, 2H, phenyl CHs; 7.08-7.27, m, 2H, phenyl CHs; 3.81, s, 3H, COOCH₃.

### d) 3-(4-Fluorophenyl)-isoxazole-4-carboxylic acid (Compound 8D)

A mixture of 0.79 g of Compound 8C, 3.5 ml of acetic acid and 3.05 ml of 37% HCl was refluxed for 4 h. After cooling to room temperature, the solvent was evaporated to dryness at reduced pressure and the crude was taken up with 15 ml of water. The precipitate was filtered off and dried to give 0.632 g (85.9%) of the title compound. M.p. 170-174°C.
^{**1**}**H-NMR (200MHz, CDCl**_{**3**}**, δ):** 13.11-13.22, br, 1H, COOH; 9.67, s, 1H, isoxazole H5; 7.70-7.89, m, 2H, phenyl CHs; 7.16-7.42, m, 2H, phenyl CHs.

### e) 3-(4-Fluorophenyl)-N-{3-[4-[4-fluoro-2-(2,2,2-trifluoroethoxy)-phenyl]-1-piperazinyl]-propyl}-isoxazole-4-carboxamide

A solution of 0.3 g of Compound 8D in 1.05 ml of thionyl chloride was refluxed for 1 h under N₂ atmosphere. After cooling to room temperature, the solvent was evaporated to dryness and the so obtained crude 3-(4-fluorophenyl)-isoxazole-4-carbonyl chloride dissolved in 10 ml of chloroform. Afterwards a solution of 0.48 g of Compound 8B and 0.6 ml of triethylamine in 10 ml of chloroform was added and the resulting mixture stirred at room temperature for 18 h. The solvent was washed with water, dried (sodium sulphate), filtered and evaporated *in vacuo*. The crude was purified by flash chromatography (ethyl acetate:2 N ammonia in methanol 95:5) to give 0.607 g (79.8%) of the title compound. M.p. 122.4-123°C.
^{**1**}**H-NMR (200MHz, CDCl**_{**3**}**, δ):** 8.89, 3, 1H, isoxazole H5; 7.74-7.87, m, 2H, trifluoroethoxyphenyl CHs; 7.28-7.41, br, 1H, NH; 7.09-7.26, m, 2H, fluorophenyl CHs; 6.71-6.91, m, 2H, fluorophenyl CHs, 6.59-6.71, m, 1H, trifluoroethoxyphenyl CH; 4.39, q, 2H, OCH₂CF₃; 3.50, q, 2H, NHCH₂; 2.83-3.01, m, 4H, 2 piperazine CH₂s; 2.48-2.72, m, 6H, CONHCH₂CH₂CH₂N and 2 piperazine CH₂s; 1.80, q, 2H, CH₂CH₂CH₂.

### Example 9

### 3-(2-Chloro-6-fluorophenyl)-N-{3-[4-[4-fluoro-2-(2,2,2-trifluoroethoxy)-phenyl]-1-piperazinyl]-propyl}-5-methylisoxazole-4-carboxamide

The title compound was synthesized following the procedure described in Example 8, but substituting commercial 3-(2-chloro-6-fluorophenyl)-5-methylisoxazole-4-carbonyl chloride for the crude 3-(4-fluorophenyl)-isoxazole-4-carbonyl chloride. Purification by flash chromatography (chloroform:2 N ammonia in methanol 100:3) afforded the title compound (67.5%) as a vitreous solid.
^{**1**}**H-NMR (200 MHz, CDCl**_{**3**}**, δ):** 7.30-7.60, m, 2H, chlorophenyl H3 and H5; 7.10-7.30, m, 1H, chlorophenyl H4; 6.58-6.98, m, 3H, fluorophenyl H3, H5 and H6; 5.80-6.55, br, 1H, NH; 4.38, q, 2H, OCH₂CF₃; 3.25-3.50, m, 2H NHCH₂; 2.90-3.25, m, 4H, 2 piperazine CH₂s; 2.30-2.90, m, 9H, CONHCH₂CH₂CH₂, CH₃, 2 piperazine CH₂s; 1.40-1.95, m, 2H, CH₂CH₂CH₂.

### Example 10

### 3-(2,6-Dichlorophenyl)-N-{3-[4-[4-fluoro-2-(2,2,2-trifluoroethoxy)-phenyl]-1-piperazinyl]-propyl}-5-methylisoxazole-4-carboxamide.

The title compound was synthesized according to the procedure described in Example 1, but using 3-(2,6-dichlorophenyl)-5-methylisoxazole-4-carboxylic acid instead of 3-phenyl-5-methyl-isoxazole-4-carboxylic acid and Compound 8B instead of Compound 1B. Extraction with diethyl ether followed by purification by flash chromatography (chloroform:2 N ammonia in methanol 100:3) afforded the title compound (38%) as an oil.
^{**1**}**H-NMR (200MHz, CDCl**_{**3**}**, δ):** 7.35-7.58, m, 3H, dichlorophenyl H3, H4 and H5; 6.58-6.93, m, 4H, fluorophenyl H3, H5 and H6; 5.35-5.72, br, 1H, NH; 4.38, q, 2H, OCH₂CF₃; 3.22-3.42, m, 2H, NHCH₂; 2.90-3.10, m, 4H, 2 piperazine CH₂s; 2.80, s, 3H, CH₃; 2.40-2.65, m, 4H, 2 piperazine CH₂s; 2.15-2.35, m, 2H, CONHCH₂CH₂CH₂N; 1.45-1.80, m, 2H, CH₂CH₂CH₂.

### Example 11

### 3-(2-Chlorophenyl)-N-{3-[4-[4-fluoro-2-(2,2,2-trifluoroethoxy)-phenyl-1-piperazinyl]-propyl}-5-methylisoxazole-4-carboxamide

The title compound was synthesized following the procedure described in Example 8, but substituting commercial 3-(2-chlorophenyl)-5-methylisoxazole-4-carbonyl chloride for the crude 3-(4-fluorophenyl)-isoxazole-4-carbonyl chloride. Purification by flash chromatography (chloroform:2 N ammonia in methanol 100:3) afforded the title compound (78.4%) as a vitreous solid.
^{**1**}**H-NMR (200MHz, CDCl**_{**3**}**, δ):** 7.38-7.62, m, 4H, chlorophenyl CHs; 6.55-6.95, m, 3H, fluorophenyl CHs; 5.40-5.80, br, 1H, NH; 4.35, q, 2H, OCH₂CF₃; 3.15-3.60, m, 2H, NHCH₂; 2.82-3.15, m, 4H, 2 piperazine CH₂s; 2.78, s, 3H, CH₃; 2.35-2.63, m, 4H, 2 piperazine CHₛs; 2.10-2.35, m, 2H, CONHCH₂CH₂CH₂N; 1.40-1.70, m, 2H, CH₂CH₂CH₂.

### Example 12

### 3-(2-Chloro-6-fluorophenyl)-5-methyl-N-{3-[4-[2-(2,2,2-trifluoroethoxy)phenyl)-1-piperazinyl]-propyl}-isoxazole-4-carboxamide

### a) 1-[2-(2,2,2-Trifluoroethoxy)-phenyl]-4-[3-(N-phthalimido)-propyl]-piperazine (Compound 12A)

The title compound was synthesized following the procedure described for Compound 1A of Example 1 but substituting 1-[2-(2,2,2-trifluoroethoxy)-phenyl]-piperazine for 1-(5-chloro-2-methoxyphenyl)-piperazine. The reaction mixture was extracted with diethyl ether and the organic solution was dried over sodium sulphate and filtered on silica gel, washing the panel with diethyl ether. Evaporation to dryness *in vacuo* afforded the title product (91%). M.p. 111-113°C.
^{**1**}**H-NMR (200MHz, CDCl**_{**3**}**, δ):** 7.60-7.92, m, 4H, phthalimide CHs; 6.80-7.10, m, 4H, trifluoroethoxyphenyl CHs; 4.35, q, 2H, OCH₂CF₃; 3.80, t, 2H, (CO)₂NCH₂; 2.75-3.12, m, 4H, 2 piperazine CH₂s; 2.30-2.75, m, 6H, (CO)₂NCH₂CH₂CH₂N, 2 piperazine CH₂s; 1.75-2.10, m, 2H, CH₂CH₂CH₂.

### b) 1-(3-Aminopropyl)-4-[2-(2,2,2-trifluoroethoxy)-phenyl]-piperazine (Compound 12B)

The title compound was synthesized following the procedure described for Compound 1B of Example 1 but substituting Compound 12A for Compound 1A. Extraction of alkalinized filtrate with dichloromethane followed by purification by flash chromatography (chloroform: 2N ammonia methanol 10:1 ) afforded the title compound (78%) as an oil.
^{**1**}**H-NMR (200MHz, CDCl**_{**3**}**, δ):** 6.82-7.12, m, 4H, aromatic CHs; 4.40, q, 2H, OCH₂CF₃; 2.95-3.25, m, 4H, 2 piperazine CH₂s; 2.72-2.85, m, 2H, H₂NCH₂CH₂CH₂N; 2.52-2.72, m, 2 piperazine CH₂s; 2.38-2.52, m, 2H, H₂NCH₂CH₂CH₂N; 1.55-1.80, m, 4H, H₂NCH₂CH₂CH₂N.

### c) 3-(2-Chloro-6-fluorophenyl)-5-methyl-N-(3-(4-(2-(2,2,2-trifluoroethoxy)-phenyl)-1-piperazinyl]-propyl}-isoxazole-4-carboxamide

The title compound was synthesized according to the procedure described in Example 8 but using 3-(2-chloro-6-fluorophenyl)-5-methylisoxazole-4-carbonyl chloride instead of the crude 3-(4-fluorophenyl)-isoxazole-4-carbonyl chloride and Compound 12B instead of Compound 8B. Washing the reaction mixture with 2 N sodium hydroxide, followed by the usual work-up and purification by flash chromatography (chloroform:2 N ammonia in methanol 100:3) afforded the title compound (92%) as an oil.
^{**1**}**H-NMR (200MHz, CDCl**_{**3**}**, δ):** 7.30-7.55, m, 2H, chlorophenyl H3 and H5; 7.10-7.23, m, 1H, chlorophenyl H4; 6.80-7.10, m, 4H, trifluoroethoxyphenyl CHs; 5.60-6.30, br, 1H, NH; 4.40, q, 2H, OCH₂CF₃; 3.25-3.50, m, 2H, NHCH₂; 2.90-3.25, m, 4H, 2 piperazine CH₂s; 2.78, s, 3H, CH₃; 2.10-2.75, m, 6H, CONHCH₂CH₂CH₂N, 2 piperazine CH₂s; 1.40-1.90, m, 2H, CH₂CH₂CH₂.

### Example 13

### (2-Chlorophenyl)-5-methyl-N-{3-[4-[2-(2,2,2-trifluoroethoxy)-phenyl]-1-piperazinyl]-propyl}-isoxazole-4-carboxamide

The title compound was synthesized according to the procedure described in Example 8, but using 3-(2-chlorophenyl)-5-methylisoxazole-4-carbonyl chloride instead of the crude 3-(4-fluorophenyl)-isoxazole-4-carbonyl chloride and Compound 12B instead of Compound 8B. Washing the reaction mixture with 2 N sodium hydroxide followed by the usual work-up and purification by flash chromatography (chloroform:2 N ammonia in methanol 100:3) afforded the title compound (67.2%) as an oil.
^{**1**}**H-NMR (200MHz, CDCl**_{**3**}**, δ):** 7.35-7.62, m, 4H, chlorophenyl CHs; 6.82-7.12, m, 4H, trifluoroethoxyphenyl CHs; 5.50-5.80, br, 1H, NH; 4.40, q, 2H, OCH₂CF₃; 3.22-3.42, m, 2H, NHCH₂; 2.88-3.15, m, 4H, 2 piperazine CH₂s; 2.78, s, 3H, CH₃; 2.35-2.63, m, 4H, 2 piperazine CH₂s; 2.10-2.35, m, 2H, CONHCH₂CH₂CH₂N; 1.45-1.75, m, 2H, CH₂CH₂CH₂.

### Example 14

### (2,6-Dichlorophenyl)-5-methyl-N-{3-[4-[2-(2,2,2-trifluoroethoxy)-phenyl]-1-piperazinyl]-propyl}-isoxazole-4-carboxamide

The title compound was synthesized according to the procedure described in Example 1 but using 3-(2,6-dichlorophenyl)-5-methylisoxazole-4-carboxylic acid instead of 3-phenyl-5-methylisoxazole-4-carboxylic acid and Compound 12B instead of Compound 1B. Extraction with diethyl ether followed by purification by flash chromatography (chloroform:2 N ammonia in methanol 100:3) afforded the title compound (75%) as an oil.
^{**1**}**H-NMR (200MHz, CDCl**_{**3**}**, δ):** 7.35-7.60, m, 3H, dichlorophenyl H3, H4 and H5; 6.82-7.12, m, 4H, trifluoroethoxyphenyl CHs; 5.40-5.75, br, 1H, NH; 4.40, q, 2H, OCH₂CF₃; 3.22-3.42, m, 2H, NHCH₂; 2.98-3.18, m, 4H, 2 piperazine CH₂s; 2.80, s, 3H, CH₃; 2.40-2.65, m, 4H, 2 piperazine CH₂s; 2.12-2.35, m, 2H, CONHCH₂CH₂CH₂N; 1.45-1.75, m, 2H, CH₂CH₂CH₂.

### Example 15

### N-{3-[4-[4-Fluoro-2-{2,2,2-trifluoroethoxy}-phenyl]-1-piperazinyl]-propyl}-3-phenylisoxazole-4-carboxamide

### a) Methyl 3-phenyl-4-isoxazolecarboxylate (Compound 15A)

This compound was synthesized following the procedure described for Compound 8C but using benzaldoxime instead of 4-fluorobenzaldoxime as a starting material. The crude was purified twice by flash chromatography (toluene:acetone 96:4 followed by toluene) to give the title compound as an oil (35%).
^{**1**}**H-NMR (200MHz), CDCl**_{**3**}**, δ):** 9.01, s, 1H, isoxazole H5; 7.69-7.84, m, 2H, phenyl CHs; 7.41-7.56, m, 3H, phenyl CHs; 3.87, s, 3H, COOCH₃.

### b) 3-Phenyl-4-isoxazolecarboxylic acid (Compound 15B)

This compound was synthesized following the procedure described for Compound 8D but using Compound 15A instead of Compound 8C (90.2%). M.p. 162.7-164.5°C.
^{**1**}**H-NMR (200MHz, DMSO-d6, δ):** 13.07-13.23, br, 1H, COOH; 9.65, s, 1H, isoxazole H5; 7.68-7.78, m, 2H, phenyl CHs; 7.41-7.58, m, 3H, phenyl CHs.

### c) N-{3-[4-[4-Fluoro-2-(2,2,2-trifluoroethoxy)-phenyl-1-piperazinyl]-propyl}-3-phenylisoxazole-4-carboxamide

The title compound was synthesized following the method described in Example 8 but substituting Compound 15B for Compound 8D. The crude was purified by flash chromatography (ethyl acetate:2 N ammonia in methanol 95:5) to give the title compound (88.4%). M.p. 95.8°C.
^{**1**}**H-NMR (200MHz, CDCl**_{**3**}**, δ):** 9.09, s, 1H, isoxazole H5; 7.69-7.81, m, 2H, phenyl CHs; 7.44-7.66, m, 4H, phenyl CHs and NH; 6.84-6.95, m, 1H, phenyl CH; 6.69-6.82, m, 1H, phenyl CH; 6.58-6.67, m, 1H, phenyl CH; 4.39, q, 2H, OCH₂CF₃; 3.51, q, 2H, NHCH₂; 3.03-3.25, m, 4H, 2 piperazine CH₂s; 2.79-2.97, m, 4H, 2 piperazine CH₂s; 2.73, t, 2H, CONHCH₂CH₂CH₂N; 1.91, dt, 2H, CH₂CH₂CH₂.

### Example 16

### 3-(4-Fluorophenyl)-N-{3-[4-[2-(2,2,2-trifluoroethoxy)-phenyl]-1-piperazinyl]-propyl}-isoxazole-4-carboxamide

The title compound was prepared according to the procedure described in Example 8, but substituting compound 12B for Compound 8B. Purification by flash chromatography (ethyl acetate:2 N ammonia in methanol 95:5) yielded the title product: 91.7%. M.p. 86.5-87.5°C.
^{**1**}**H-NMR (200MHz, CDCl**_{**3**}**, δ):** 8.84, s, 1H, isoxazole H5; 7.31-7.40, br, 1H, NH; 7.72-7.87, m, 2H, trifluoroethoxyphenyl CHs; 7.10-7.27, m, 2H, trifluoroethoxyphenyl CHs; 6.96-7.08, m, 2H, fluorophenyl CHs; 6.81-6.94, m, 2H, fluorophenyl CHs; 4.39, q, 2H, OCH₂CF₃; 3.50, q, 2H, NHCH₂; 2.83-3.08, m, 4H, 2 piperazine CH₂s; 2.53-2.64, m, 4H, 2 piperazine CH₂s; 2.49, t, 2H, CONHCH₂CH₂CH₂N; 1.74, dt, 2H, CH₂CH₂CH₂.

### Example 17

### 3-Phenyl-N-{3-[4-[2-(2,2,2-trifluoroethoxy)-phenyl]-1-piperazinyl]-propyl}-isoxazole-4-carboxamide

The title compound was prepared according to the procedure described in Example 8, but substituting compound 15B for Compound 8D and Compound 12B for Compound 8B. Purification by flash chromatography (ethyl acetate:2 N ammonia in methanol 95:5) yielded the title product (88%). M.p. 94.5-95.6°C.
^{**1**}**H-NMR (200MHz, CDCl**_{**3**}**, δ):** 8.87, s, 1H, isoxazole H5; 7.67-7.85, m, 2H, phenyl CHs; 7.43-7.58, m, 3H, phenyl CHs; 6.84-7.12, m, 5H, NH and 4 trifluoroethoxyphenyl CHs; 4.39, q, 2H, OCH₂CF₃; 3.46, q, 2H, NHCH₂; 2.88-3.02, m, 4H, 2 piperazine CH₂s; 2.47-2.59, m, 4H, 2 piperazine CH₂s; 2.42, t, 2H, CONHCH₂CH₂CH₂N; 1.81, q, 2H, CH₂CH₂CH₂.

### Example 18

### 3-(4-Fluorophenyl)-N-{3-[3-[4-fluoro-2-(2,2,2-trifluoroethoxy)-phenyl]-1-piperazinyl]-propyl}-5-methylisoxazole-4-carboxamide

The title compound was prepared according to the procedure described in Example 1, but substituting Compound 8B for Compound 1B and 3-(4-fluorophenyl)-5-methylisoxazole-4-carboxylic acid (described in *J.Chem.Soc*. 1963, 5838-5845 but prepared following the method detailed in *J.Am.Chem.Soc*. 1985, 107, 2721-2730) for 5-methyl-3-phenylisoxazole-4-carboxylic acid. Purification by flash chromatography (ethyl acetate:2 N ammonia in methanol 96:4) yielded the title product (77%). M.p. 150-151°C.
^{**1**}**H-NMR (200MHz, CDCl**_{**3**}**, δ):** 7.60-7.65, m, 2H, fluorophenyl CHs; 7.10-7.25, m, 2H, fluorophenyl CHs; 6.70-6.90, m, 2H, trifluoroethoxyphenyl H3, H6; 6.55-6.65, m, 1H, trifluoroethoxyphenyl H5; 6.50, bs, 1H, NH; 4.30-4.45, m, 2H, OCH₂CF₃; 3.45, q, 2H NHCH₂; 2.75-2.95, m, 4H, 2 piperazine CH₂s; 2.65, s, 3H, CH₃; 2.30-2.60, m, 6H, 2 piperazine CH₂s and CONHCH₂CH₂CH₂N; 1.50-1.75, m, 2H, CH₂CH₂CH₂.

### Example 19

### 5-Ethyl-3-phenyl-N-{3-[4-[2-(2,2,2-trifluoroethoxy)-phenyl)-1-piperazinyl]-propyl}-isoxazole-4-carboxamide

### a) N-(3-Chloropropyl)-5-ethyl-3-phenylisoxazole-4-carboxamide (Compound 19A)

A solution of 3.1 g of 5-ethyl-3-phenylisoxazole-4-carboxylic acid (prepared as described in *J. Org. Chem*. 1985, *50,* 5660-5666), 2.94 g of 3-chloropropylamine hydrochloride, 2.62 ml of 92% diethyl cyanophosphonate and 4.38 ml of triethylamine in 50 ml of anhydrous dimethylformamide was stirred at room temperature for 3 hours, poured into 500 ml of water and extracted with ethyl acetate (2 × 200 ml). The combined organic layers were washed with water and dried (sodium sulphate), and the solvent was evaporated off under vacuum. The crude was purified by flash chromatography (ethyl acetate:petroleum ether 3:7) to give 3.35 g (73%) of the title compound. M.p. 75-76°C.
^{**1**}**H-NMR (200MHz, CDCl**_{**3,**} **δ):** 7.42-7.62, m, 5H, phenyl CHs; 5.50, bs, 1H, NH; 3.15, q, 2H, CH₂CH₃; 3.31-3.45, m, 4H, CH₂CH₂CH₂Cl; 1.87, tt, 2H, CH₂CH₂CH₂; 1.38, t, 3H, CH₃.

### b) 5-Ethyl-3-phenyl-N-{3-[4-[2-(2,2,2-trifluoroethoxy)-phenyl]-1-piperazinyl]-propyl}-isoxazole-4-carboxamide

The title compound was obtained following the procedure described in Example 2 but substituting Compound 19A for Compound 2B and 1-[2-(2,2,2-trifluoroethoxy)-phenyl]-piperazine for 1-(2-hydroxy-5-chlorophenyl)-piperazine. The crude was taken up with ethyl acetate and purified by flash chromatography eluting (ethyl acetate:2 N ammonia in methanol 98:2) to afford the title compound (54%). M.p. 102-104°C.
^{**1**}**H-NMR (200MHz, CDCl**_{**3**}**; δ):** 7.58-7.72, m, 3H, phenyl CHs, 7.39-7.57, m, 2H, phenyl CHs; 6.82-7.12, m, 4H, trifluoroethoxyphenyl CHs; 6.40, bs, 1H, NH; 4.36; q, 2H, CH₂CF₃; 3.39, dt, 2H, NHCH₂; 3.10, q, 2H, CH₂CH₃; 2.71-3.01, m, 4H, 2 piperazine CH₂s; 2.20-2.59, m, 6H, 2 piperazine CH₂s and CONHCH₂CH₂CH₂N; 1.52-1.75, m, 2H, CH₂CH₂CH₂; 1.38, t, 3H, CH₃.

### Example 20

### 5-Ethyl-N-{3-[4-[4-fluoro-2-(2,2,2-trifluoroethoxy)-phenyl]-1-piperazinyl]-propyl}-3-phenylisoxazole-4-carboxamide

The title compound was obtained following the procedure described in Example 2 but substituting Compound 19A for Compound 2B and 1-[4-fluoro-2-(2,2,2-trifluoroethoxy)-phenyl]-piperazine for 1-(2-hydroxy-5-chlorophenyl)-piperazine. The crude was taken up with ethyl acetate and purified by flash chromatography (ethyl acetate:2 N ammonia in methanol 98:2) to afford the title compound (64%). M.p. 115-117°C.
^{**1**}**H-NMR (200MHz, CDCl**_{**3**}**, δ):** 7.58-7.72, m, 3H, phenyl CHs; 7.39-7.55, m, 2H, phenyl CHs; 6.68-6.88, m, 2H, trifluoroethoxyphenyl H3 and H6; 6.60, dd, 1H, trifluoroethoxyphenyl H5; 6.35, bs, 1H, NH; 4.34, q, 2H, CH₂CF₃; 3.39, dt, 2H, NHCH₂; 3.10, q, 2H, CH₂CH₃; 2.68-3.01, m, 4H, 2 piperazine CH₂s; 2.15-2.54, m, 6H, 2 piperazine CH₂s and CONHCH₂CH₂CH₂N; 1.49-1.72, m, 2H, CH₂CH₂CH₂; 1.38, t, 3H, CH₃; 1.81, dt, 2H, CH₂CH₂CH₂.

### Example 21

### 3-(4-Fluorophenyl)-5-methyl-N-{3-[4-[2-(2,2,2-trifluoroethoxy)-phenyl]-1-piperazinyl]-propyl}-isoxazole-4-carboxamide

The title compound was prepared according to the procedure described in Example 1, but substituting Compound 12B for Compound 1B and 3-(4-fluorophenyl)-5-methylisoxazole-4-carboxylic acid for 5-methyl-3-phenylisoxazole-4-carboxylic acid. Purification by flash chromatography (ethyl acetate:2 N ammonia in methanol 97:3) yielded the title product (66%). M.p. 132-134°C.
^{**1**}**H-NMR (200MHz, CDCl**_{**3**}**; δ):** 7.60-7.65, m, 2H, fluorophenyl CHs; 7.10-7.25, m, 2H, fluorophenyl CHs; 6.85-7.05, m, 4H, trifluoroethoxyphenyl CHs; 6.60, bs, 1H, NH; 4.30-4.45, m, 2H, OCH₂CF₃; 3.45, q, 2H, NHCH₂; 2.85-3.00, m, 4H, 2 piperazine CH₂s; 2.65, s, 3H, CH₃; 2.30-2.60, m, 6H, 2 piperazine CH₂s and CONHCH₂CH₂CH₂N; 1.60-1.80, m, 2H, CH₂CH₂CH₂.

### Example 22

### 3-(2-Fluorophenyl)-N-{3-[4-[4-fluoro-2-(2,2,2-trifluoroethoxy)-phenyl]-1-piperazinyl]-propyl}-5-methylisoxazole-4-carboxamide

The title compound was prepared according to the procedure described in Example 12, but substituting Compound 8B for Compound 1B and 3-(2-fluorophenyl)-5-methylisoxazole-4-carboxylic acid (described in *J. Chem. Soc*. 1963, 5838-5845 but prepared following the method described in *J. Am. Chem. Soc*. 1985, 107, 2721-2730)) for 5-methyl-3-phenylisoxazole-4-carboxylic acid. Purification by flash chromatography (ethyl acetate:2 N ammonia in methanol 94:6) yielded the title product (83%). M.p. 110-112°C.
^{**1**}**H-NMR (200MHz, CDCl**_{**3**}**, δ):** 7.50-7.70, m, 2H, fluorophenyl CHs; 7.15-7.35, m, 2H, fluorophenyl CHs; 6.75-6.90, m, 2H, trifluoroethoxyphenyl H3, H6; 6.60-6.70, m, 1H, trifluoroethoxyphenyl H5; 6.20, bs, 1H, NH; 4.40, q, 2H, OCH₂CF₃; 3.40, q, 2H, NHCH₂; 2.85-3.00, m, 4H, 2 piperazine CH₂s; 2.70, s, 3H, CH₃; 2.30-2.60, m, 6H, 2 piperazine CH₂s and CONHCH₂CH₂CH₂N; 1.50-1.75, m, 2H, CH₂CH₂CH₂.

### Example 23

### 3-(2-Fluorophenyl)-5-methyl-N-{3-[4-[2-(2,2,2-trifluoroethoxy)-phenyl]-1-piperazinyl]-propyl}-isoxazole-4-carboxamide

The title compound was prepared according to the procedure described in Example 1, but substituting Compound 12B for Compound 1B and 3-(2-fluorophenyl)-5-methylisoxazole-4-carboxylic acid for 5-methyl-3-phenylisoxazole-4-carboxylic acid. Purification by flash chromatography (ethyl acetate:2 N ammonia in methanol 96:4) yielded the title product (61%). M.p. 127-128°C.
^{**1**}**H-NMR (200MHz, CDCl**_{**3**}**, δ):** 7.45-7.65, m, 2H, fluorophenyl CHs; 7.15-7.35, m, 2H, fluorophenyl CHs; 6.90-7.10, m, 4H, trifluoroethoxyphenyl CHs; 6.30, bs, 1H, NH; 4.40, m, 2H, OCH₂CF₃; 3.40, q, 2H, NHCH₂; 2.90-3.10, m, 4H, 2 piperazine CH₂s; 2.70, s, 3H, CH₃; 2.30-2.60, m, 6H, 2 piperazine CH₂s and CONHCH₂CH₂CH₂N; 1.60-1.80, m, 2H, CH₂CH₂CH₂.

### Example 24

### 3-(3-Fluorophenyl)-N-{3-[4-[4-fluoro-2-(2,2,2-trifluoroethoxy)-phenyl]-1-piperazinyl]-propyl}-5-methylisoxazole-4-carboxamide

The title compound was prepared according to the procedure described in Example 1, but substituting Compound 8B for Compound 1B and 3-(3-fluorophenyl)-5-methylisoxazole-4-carboxylic acid (described in *J. Chem. Soc*. 1963, 5845-5854 but prepared following the method detailed in *J. Am. Chem. Soc*. 1985, 107, 2721-2730) for 5-methyl-3-phenylisoxazole-4-carboxylic acid. Purification by flash chromatography (ethyl acetate:2 N ammonia in methanol 95:5) yielded the title product (93%). M.p. 86-91°C.
^{**1**}**H-NMR (200MHz, CDCl**_{**3**}**, δ):** 7.35-7.50, m, 3H, fluorophenyl CHs; 7.15-7.30, m, 1H, fluorophenyl CH; 6.70-6.90 m, 2H, trifluoroethoxyphenyl H3, H6; 6.55-6.70, m, 1H, trifluoroethoxyphenyl H5; 6.55, bs, 1H, NH; 4.37, q, 2H, OCH₂CF₃; 3.45, q, 2H, NHCH₂; 2.70-2.95, m, 4H, 2 piperazine CH₂s; 2.65, s,. 3H, CH₃; 2.30-2.60, m, 6H, 2 piperazine CH₂s and CONHCH₂CH₂CH₂N; 1.50-1.75, m, 2H, CH₂CH₂CH₂.

### Example 25

### 3-(3-Fluorophenyl)-5-methyl-N-{3-[4-[2-(2,2,2-trifluoroethoxy)-phenyl]-1-piperazinyl]-propyl}-isoxazole-4-carboxamide

The title compound was prepared according to the procedure described in Example 1, but substituting Compound 12B for Compound 1B and 3-(3-fluorophenyl)-5-methylisoxazole-4-carboxylic acid for 5-methyl-3-phenylisoxazole-4-carboxylic acid. Purification by flash chromatography (ethyl acetate:2 N ammonia in methanol 96:4) yielded the title product (53%). M.p. 129-130°C.
^{**1**}**H-NMR (200MHz, CDCl**_{**3**}**, δ):** 7.35-7.50, m, 3H, fluorophenyl CHs; 7.15-7.30, m, 1H, fluorophenyl CH; 6.85-7.10, m, 4H, trifluoroethoxyphenyl CHs; 6.60, bs, 1H, NH; 4.40, m, 2H, OCH₂CF₃; 3.45, q, 2H, NHCH₂; 2.80-3.00, m, 4H, 2 piperazine CH₂s; 2.70, s, 3H, CH₃; 2.30-2.60, m, 6H, 2 piperazine CH₂s and CONHCH₂CH₂CH₂N; 1.60-1.80, m, 2H, CH₂CH₂CH₂.

### Example 26

### 3-(4-Methoxyphenyl)-5-methyl-N-{3-{4-[2-(2,2,2-trifluoroethoxy)-phenyl]-1-piperazinyl]-propyl)-isoxazole-4-carboxamide

The title compound was prepared according to the procedure described in Example 1, but substituting Compound 12B for Compound 1B and 3-(4-methoxyphenyl)-5-methylisoxazole-4-carboxylic acid (*J. Chem. Soc*. 1963, 5838-5945) for 5-methyl-3-phenylisoxazole-4-carboxylic acid. Purification by flash chromatography (ethyl acetate:2 N ammonia in methanol 96:4) yielded the title product (60%). M.p. 130-135°C.
^{**1**}**H-NMR (200MHz, CDCl**_{**3**}**, δ):** 7.55, dd, 2H, methoxyphenyl H2, H6; 6.85-7.10, m, 6H, trifluoroethoxyphenyl CHs and methoxyphenyl H3, H5; 6.35, t, 1H, NH; 4.35, q, 2H, OCH₂CF₃; 3.85, s, 3H, OCH₃; 3.40, q, 2H, NHCH₂; 2.80-3.00, m, 4H, 2 piperazine CH₂s; 2.65, s, 3H, CH₃; 2.30-2.50, m, 6H, 2 piperazine CH₂s and CONHCH₂CH₂CH₂N; 1.55-1.75, m, 2H, CH₂CH₂CH₂.

### Example 27

### N-{3-[4-[4-Fluoro-2-(2,2,2-trifluoroethoxy)-phenyl]-1-piperazinyl]-propyl}-3-(4-methoxyphenyl)-5-methylisoxazole-4-carboxamide

This title compound was prepared according to the procedure described in Example 1, but substituting Compound 8B for Compound 1B and 3-(4-methoxyphenyl)-5-methylisoxazole-4-carboxylic acid for 5-methyl-3-phenylisoxazole-4-carboxylic acid. Purification by flash chromatography (ethyl acetate:2 N ammonia in methanol 95:5) yielded the title product (78%). M.p. 106-109°C.
^{**1**}**H-NMR (200MHz, CDCl**_{**3**}**, δ):** 7.55, dd, 2H, methoxyphenyl H2, H6;
7.00, dd, 2H, methoxyphenyl H3, H5; 6.55-6.90, m, 3H, trifluoroethoxyphenyl CHs; 6.35, t, 1H, NH; 4.35, q, 2H, OCH₂CF₃; 3.85, s, 3H, OCH₃; 3.40, q, 2H, NHCH₂; 2.75-2.90, m, 4H, 2 piperazine CH₂s; 2.65, s, 3H, CH₃; 2.30-2.50, m, 6H, 2 piperazine CH₂s and CONHCH₂CH₂CH₂N; 1.55-1.70, m, 2H, CH₂CH₂CH₂

### Example 28

### N-{3-[4-[4-Fluoro-2-(2,2,2-trifluoroethoxy)-phenyl]-1-piperazinyl]-propyl}-3-phenyl-5-(2-phenylethyl)-isoxazole-4-carboxamide

### a) 3-Phenyl-5-(2-phenylethyl)-isoxazole-4-carboxylic acid (Compound 28A)

To a solution of 1.5 g of 5-methyl-3-phenyl-4-isoxazolecarboxylic acid in 50 ml of anhydrous tetrahydrofuran stirred at -78°C under N₂ atmosphere, 5.9 ml of a 2.5M solution of n-butyl lithium in n-hexane was added and the solution was stirred at -78°C for 2 hours. Afterwards, 0.89 ml of benzyl bromide was added at -78°C and the solution stirred at room temperature for 2 hours. After overnight resting, the solution was poured into water (300 ml), acidified with 1N hydrochloric acid and extracted with ethyl acetate (2 × 200 ml). The combined organic layers were washed with water, dried (sodium sulphate) and the solvents were evaporated to dryness. The solid residue was washed with petroleum ether to give 1.82 g (84%) of the title compound as an amorphous solid.
^{**1**}**H-NMR (200MHz, CDCl**_{**3**}**, δ):** 9.20-11.80, br, 1H, COOH; 7.10-7.90, m, 10H, phenyl CHs; 3.45, t, 2H, CH₂CH₂Ph; 3.10, t, 2H, CH₂CH₂Ph.

### b) N-{3-[4-[4-Fluoro-2-(2,2,2-trifluoroethoxy)-phenyl]-1-piperazinyl]-propyl}-3-phenyl-5-(2-phenylethyl)-isoxazole-4-carboxamide

The title compound was prepared according to the procedure described in Example 1, but substituting Compound 8B for Compound 1B and Compound 28A for 5-methyl-3-phenylisoxazole-4-carboxylic acid. Purification by flash chromatography (ethyl acetate:methanol 99:1) yielded the title product (32%) as a yellow oil.
^{**1**}**H-NMR (200MHz, CDCl**_{**3**}**, δ):** 7.58-7.71, m, 2H, 3-phenyl H2 and H6; 7.48-7.57, m, 3H, 3-phenyl H3, H4 and H5; 7.12-7.48, m, 5H, phenylethyl CHs; 6.71-6.88, m, 2H, fluorophenyl H3 and H6; 6.60, dd, 1H, fluorophenyl H5; 6.00, bt, 1H, NH; 4.38, q, 2H, CH₂CF₃; 3.22-3.41, m, 4H, CONHCH₂CH₂CH₂N and CH₂CH₂Ph; 3.08, t, 2H, CH₂CH₂Ph; 2.68-2.91, m, 4H, 2 piperazine CH₂s; 2.35-2.51, m, 4H, 2 piperazine CH₂s; 2.25, t, 2H, CONHCH₂CH₂CH₂N; 1.55, tt, 2H, CONHCH₂CH₂CH₂N

### Example 29

### N-{3-[4-[4-Fluoro-2-(2,2,2-trifluoroethoxy)-phenyl]-1-piperazinyl]-propyl}-3-phenyl-5-(3-phenylpropyl)-isoxazole-4-carboxamide

### a) 3-Phenyl-5-(3-phenylpropyl)-isoxazole-4-carboxylic acid (Compound 29A)

The title compound was prepared as described for Compound 28A but substituting 2-phenylethyl bromide for benzyl bromide. The crude was purified by flash chromatography (ethyl acetate:petroleum ether:acetic acid 1:1:0.1) to afford the title compound (72%).
^{**1**}**H-NMR (200MHz, CDCl**_{**3**}**, δ):** 9.20-11.80, br, 1H, COOH; 7.61-7.75, m, 3H, phenyl H3, H4 and H5; 7.45-7.75, m, 2H, phenyl H2 and H6; 7.10-7.35, m, 5H, CH₂CH₂CH₂Ph; 3.20, t, 2H, CH₂CH₂CH₂Ph; 2.75, t, 2H, CH₂CH₂CH₂Ph; 2.15, tt, 2H, CH₂CH₂CH₂Ph.

### b) N-{3-[4-[4-Fluoro-2-(2,2,2-trifluoroethoxy)-phenyl]-1-piperazinyl-propyl}-3-phenyl-5-(3-phenylpropyl)-isoxazole-4-carboxamide

The title compound was prepared according to the procedure described in Example 1, but substituting Compound 8B for Compound 1B and Compound 29A for 5-methyl-3-phenylisoxazole-4-carboxylic acid. Purification by flash chromatography (ethyl acetate:petroleum ether 99:1) yielded the title product (42%) as a yellow oil.
^{**1**}**H-NMR (200MHz, CDCl**_{**3**}**, δ):** 7.60-7.75, m, 2H, isoxazole 3-phenyl H2 and H6; 7.48-7.57, m, 3H, isoxazole 3-phenyl H3, H4 and H5; 7.12-7.40, m, 5H, CH₂CH₂CH₂Ph; 6.71-6.88, m; 2H, fluorophenyl H3 and H6; 6.62, dd, 1H, fluorophenyl H5; 6.40, bt, 1H, NH; 4.38, q, 2H, CH₂CF₃; 3.48, dt, 2H, CONHCH₂CH₂CH₂N; 3.12, t, 2H, CH₂CH₂CH₂Ph; 2.76-2.91, m, 4H, 2 piperazine CH₂s; 2.75, t, 2H, CH₂CH₂CH₂Ph; 2.35-2.51, m, 4H, 2 piperazine CH₂s; 2.25, t, 2H, CONHCH₂CH₂CH₂N; 2.14, tt, 2H, CH₂CH₂CH₂Ph; 1.55, tt, 2H, CONHCH₂CH₂CH₂N.

### Example 30

### N-{3-[4-[4-Fluoro-2-methoxyphenyl]-1-piperazinyl]-propyl}-5-methyl-3-phenylisoxazole-4-carboxamide

The title compound as prepared following the procedure described in Example 2c but substituting Compound 2A with 1-(4-fluoro-2-methoxyphenyl)-piperazine (US 5358948) and heating at 190°C for 40 min. The crude was purified by flash chromatography (ethyl acetate:2N methanolic ammonia 96:4) to afford the title compound (79%). M.p. 162-163°C.
^{**1**}**H-NMR (200MHz, CDCl**_{**3**}**, δ):** 7.60-7.70, m, 2H, phenyl H2, H6; 7.45-7.55, m, 3H, phenyl H3, H4, H5; 6.55-6.70 and 6.73-6.85, 2m, 3H, methoxyphenyl H3, H5, H6; 6.35, bs, 1H, NH; 3.90, s, 3H, OCH₃; 3.37, q, 2H, CONHCH₂CH₂CH₂; 2.75-2.90, m, 4H, 2 piperazine CH₂s; 2.70, s, 3H, CH₃; 2.40-2.55, m, 4H, 2 piperazine CH₂s; 2.30, t, 2H, CONHCH₂CH₂CH₂; 1.55-1.70, m, 2H, CH₂CH₂CH₂

### Example 31

### N-{3-[4-(4-Flnoro-2-methylphenyl)-1-piperazinyl]-propyl}-5-methyl-3-phenylisoxazole-4-carboxamide

### a) 1-(4-Fluoro-2-methylphenyl)-piperazine (Compound 31A)

The title compound was prepared following the method described for Compound 5C of Example 5 but starting from 4-fluoro-2-methylaniline instead of compound 5B and using a 10:1 mixture of 1,2-dichlorobenzene and *n*-hexanol at 185°C as solvent. Purification by flash chromatography (chloroform:2 N ammonia in methanol 100:3 to 100:5) afforded the title compound (70%) as a light brown solid.
^{**1**}**H-NMR (200MHz, CDCl**_{**3**}**, δ):** 6.87-7.05, m, 3H, phenyl CHs; 3.00-3.15, m, 4H, 2 piperazine CH₂s; 2.85-2.95, m, 5H, 2 piperazine CH₂s and NH; 2.30, s, 3H, CH₃.

### b) N-{3-[4-(4-Fluoro-2-methylphenyl)-1-piperazinyl]-propyl}-5-methyl-3-phenylisoxazole-4-carboxamide

A stirred mixture of 0.380 g of Compound 31A, 0.50 ml of N,N-diisopropyl-ethylamine, 0.545 g of Compound 2B and 3ml of N,N-dimethylformamide was heated at 120°C for 5 hours. The solution was diluted with water (60 ml) and extracted with dichloromethane (3×30 ml); the organic layer was washed with water (3×20 ml), dried (anhydrous sodium sulphate) and evaporated to dryness *in vacuo*. The residue was purified by flash chromatography (chloroform:2 N methanolic ammonia 100:1) affording 0.373 g (43.7%) of the title compound as an ivory solid. M.p. 139-141°C.
^{**1**}**H-NMR (200MHz, CDCl**_{**3**}**, δ):** 7.58-7.73, m, 2H, phenyl H2, H6; 7.42-7.58, m, 3H, phenyl H3, H4, H5; 6.77-6.97, m, 3H, fluorophenyl CHs; 6.35, bs, 1H, CONH; 3.30-3.50, m, 2H, CONHCH₂CH₂CH₂; 2.58-2.75, m, 7H, piperazine CH₂s, isoxazole CH₃; 2.18-2.50, m, 9H, CONHCH₂CH₂CH₂, phenyl CH₃, 2 piperazine CH₂s; 1.50-1.72, m, 2H, CONHCH₂CH₂CH₂.

### Example 32

### N-{3-[4-(4-Chloro-2-methylphenyl)-1-piperazinyl]-propyl}-5-methyl-3-phenylisoxazole-4-carboxamide

### a) 1-(4-Chloro-2-methylphenyl)-piperazine (Compound 32A)

The title compound was prepared following the method described for Compound 31A of Example 31 but starting from 4-chloro-2-methylaniline instead of 4-fluoro-2-methylaniline. Purification by flash chromatography (chloroform:2 N ammonia in methanol 100:3 to 100:5) afforded the title compound (73%) as an oil.
^{**1**}**H-NMR (200MHz, CDCl**_{**3**}**, δ):** 7.05-7.20, m, 2H, phenyl H3, H5; 6.95, d, 1H, phenyl H6; 2.95-3.05, m, 4H, 2 piperazine CH₂s; 2.75-2.85, m, 4H, 2 piperazine CH₂s; 2.28, s, 3H, CH₃; 1.70, s, 1H, NH

### b) N-{3-[4-(4-Chloro-2-methylphenyl)-1-piperazinyl]-propyl}-5-methyl-3-phenylisozazole-4-carboxamide

The title compound was prepared following the method described in Example 31, but using Compound 32A instead of Compound 31A. The residue was purified by flash chromatography (chloroform:2 N methanolic ammonia 100:1) affording the title compound (47%) as an ivory solid. M.p. 147-150°C.
^{**1**}**H-NMR (200MHz, CDCl**_{**3**}**, δ):** 7.58-7.75, m, 2H, phenyl H2, H6; 7.40-7.58, m, 3H, phenyl H3, H4, H5; 7.07-7.20, m, 2H, chlorophenyl H3 and H5; 6.85, d, 1H, chlorophenyl H6; 6.32, bs, 1H, CONH; 3.30-3.50, m, 2H, CONHCH₂CH₂CH₂; 2.62-2.80, m, 7H, 2 piperazine CH₂s, isoxazole CH₃; 2.18-2.52, m, 9H, CONHCH₂CH₂CH₂, phenyl CH₃, 2 piperazine CH₂s; 1.52-1.75, m, 2H, CONHCH₂CH₂CH₂.

### Example 33

### N-{3-[4-[4-Fluoro-2-(1-methylethoxy)-phenyl]-1-piperazinyl]-propyl}-5-methyl-3-phenylisoxazole-4-carboxamide

The title compound was prepared following the procedure described in Example 2c but substituting Compound 2A with 1-[4-fluoro-2-(1-methylethoxy)-phenyl]-piperazine (US 5569659) and heating at 190°C for 40 min. The crude was purified by flash chromatography (dichloromethane:2N methanolic ammonia 95:5) to afford the title compound (55%) as an ivory solid. M.p. 104-106°C.
^{**1**}**H-NMR (200MHz, CDCl**_{**3**}**, δ):** 7.60-7.70, m, 2H, phenyl H2, H6; 7.45-7.55, m, 3H, phenyl H3, H4, H5; 6.70-6.80 and 6.55-6.65, 2m, 3H, isopropoxyphenyl H3, H5, H6; 6.35, br, 1H, NH; 4.55, sept, 1H, CH; 3.40, q, 2H, CONHCH₂CH₂CH₂; 2.75-2.95, m, 4H, 2 piperazine CH₂s; 2.70, s, 3H, CH₃; 2.35-2.50, m, 4H, 2 piperazine CH₂s; 2.30, t, 2H, CONHCH₂CH₂CH₂; 1.55-1.70, m, 2H, CH₂CH₂CH₂; 1.35, d, 6H, (CH₃)₂.

### Example 34

### Determination of affinity for cloned α₁ adrenergic receptors and 5-HT_{1A} serotoninergic receptors by radioligand binding assay

Determination of affinity for cloned subtypes of the cloned α₁-adrenoceptor was performed in membranes from cells transfected by electroporation with DNA expressing the genes encoding each α₁-adrenoceptor subtype.

Cloning and stable expression of the α₁-adrenoceptor gene were performed as previously described (Testa R. et al., *Pharmacol. Comm*. 6, 79-86 (1995) and references). The cell membranes were incubated in 50 nM Tris, pH 7.4, with 0.2 nM [³H]prazosin, in a final volume of 1.02 ml for 30 minutes at 25°C, in the absence or presence of competing drugs (1 pM-10 µM). Non-specific binding was determined in the presence of 10 µM phentolamine. Incubation was stopped by addition of ice-cold Tris buffer and rapid filtration through 0.2%-polyethyleneimine pretreated Schleicher & Schuell GF52 filters. Genomic clone G-21 coding for the human 5-HT_{1A}-serotoninergic receptor was stably transfected in a human cell line (HeLa) (Fargin A. et al., *J. Biol. Chem*. 284, 14848-14852 (1989)). HeLa cells were grown as monolayers in Dulbecco's modified Eagle's medium (DMEM), supplemented with 10% foetal calf serum and gentamicin (100 µg/ml), 5% CO₂ at 37°C. The cells were detached from the growth flask at 95% confluence by a cell scraper and were lysed in ice-cold Tris-5-mM and EDTA-5-mM buffer (pH 7.4). The homogenates were centrifuged at 40000 × g × 20 minutes and the membranes were resuspended in a small volume of ice-cold buffer containing Tris 5 mM and EDTA 5 mM (pH 7.4) and immediately frozen and stored at -70°C until use.

On the day of experiment, the cell membranes were resuspended in a buffer containing 50 mM Tris (pH 7.4), 2.5 mM MgCl₂, 10 µM pargyline (Fargin A. et al., *Nature* 335, 358-360 (1988)). The membranes were incubated in a final volume of 1 ml for 30 minutes at 30°C with 1.2 nM [³H]8-OH-DPAT, in the absence or presence of test molecules. Non-specific binding was determined in the presence of 10 µM 5-HT. Incubation was stopped by addition of ice-cold Tris buffer and rapid filtration through 0.2%-polyethyleneimine pretreated Schleicher & Schuell GF52 filters.

Inhibition of specific binding of the radioligands by the test drugs was analysed to estimate the IC₅₀ value by using the non-linear curve-fitting program Allfit (De Lean A. et al., *Am. J. Physiol*. 235, E97-E102 (1978)).

The IC₅₀ value was converted to an affinity constant (Ki) by the equation of Cheng Y. C. et al., *Biochem. Pharmacol.* 22, 3099-3108 (1973). Data were expressed as mean of Ki.

### RESULTS

The compounds of the invention exhibited the desired potency and selectivity at α₁ adrenoceptors, as shown in Table 1.

**TABLE 1**

| *Affinity (Ki, nM) of the different compounds tested for recombinant α*_{*1*}*-adrenoceptor subtypes and 5-HT*_{*1A*} *receptor.* | | | | |
|---|---|---|---|---|
| **Example** | **Receptors** | | | |
| | **α**_{**1b**} | **α**_{**1b**} | **α**_{**1d**} | **5-HT**_{**1A**} |
| 1 | 0.77 | 23.64 | 2.72 | 435.66 |
| 2 | 1.16 | 9.97 | 25.16 | 49.46 |
| 3 | 0.066 | 36.21 | 1.45 | 11.72 |
| 4 | 16.99 | 569.79 | 128.43 | > 1000 |
| 5 | 0.05 | 11.52 | 0.33 | 191.74 |
| 6 | 0.35 | 69.50 | 18.89 | 316.90 |
| 7 | 0.918 | 7.49 | 38.93 | 1000.00 |
| 8 | 0.11 | 28.08 | 25.81 | 201.9 |
| 9 | 0.4 | 24.1 | 6.15 | 341.9 |
| 10 | 2.28 | 24.23 | 38.58 | 169.55 |
| 11 | 0.12 | 31.15 | 4.66 | 242.11 |
| 12 | 0.16 | 21.38 | 2.19 | 29.98 |
| Compound A | 0.65 | 3.87 | 1.51 | 4.53 |
| Prazosin | 0.54 | 0.42 | 0.23 | > 10000 |

### Example 35

### In vitro evaluation of functional antagonism for α_{1L} adrenoceptors

The functional α₁-antagonistic activity of the test compounds against noradrenaline(NA)-induced contractions of rabbit aorta pretreated with chloroethylclonidine (α_{1L} receptor) was evaluated according to the method of Testa R. et al., *J. Pharmacol. Exp. Ther*. 281, 1284-1293 (1997). Adult male New Zealand rabbits were sacrificed by cervical dislocation. The aorta was removed, placed in Krebs-Henseleit buffer and dissected free of adhering tissue. Rings were prepared from each artery (8 rings per aorta, about 4-5 mm wide) and suspended in 20 ml organ bath containing Krebs bicarbonate buffer of the following composition (mM): NaCl 112.0, KCl 5.0, CaCl₂ 2.5, KH₂PO₄ 1.0, MgSO₄ 1.2, NaHCO₃ 12.0 and glucose 11.1, and equilibrated at 37° C with 95% O₂: 5% CO₂. Desmethylimipramine (0.1 µM) and corticosterone (1 µM) to block neuronal and extraneuronal uptake of NA, (±)-propranol (1 µM) to block β adrenoceptors and yohimbine (0.1 µM) to block α₂ adrenoceptors were added to the buffer. The tissues were subject to a passive load of 2 g and the developed tension was measured using isometric transducers (Basile 7003).

The preparations were allowed to equilibrate for 60 minutes and then primed every 30 minutes with 10 µM NA for three times. The aortic rings were then incubated with the alkylating agent chloroethylclonidine (5 × 10⁻⁵M) for 30 minutes and then washed extensively three times (in 0.5 hours) before constructing the NA-concentration/response curve. After washout of NA and re-equilibration of the tissue (45 min), the drug to be tested was added and, after 30 minutes, a second cumulative-NA-concentration/response curve constructed. Each antagonist concentration was tested using 2-3 aortic rings from different rabbits.

Dose ratios (i.e., the ratio between the concentrations of noradrenaline required to produce half-maximal response in the presence and in the absence of the test antagonist) were calculated at each concentration of the compounds. The logarithm of these dose ratio -1 was plotted against the logarithm of the compound concentrations (Schild plot) to evaluate the affinity constant Kb.

When only one or two concentrations of the test compounds were utilised, the apparent Kb value was calculated using the formula: Kb = [B]/(DOSE RATIO-1), where B is the antagonist concentration.

### RESULTS

The compounds tested showed good affinity for the α_{1L} adrenoceptor subtype.

The data are expressed as pKb in Table 2.

**TABLE 2**

| *Functional affinity of the tested compounds for the α*_{*1L*} *adrenoceptor subtype* | |
|---|---|
| **Example** | **pKb** |
| 3 | 8.47 |
| 5 | 8.79 |
| Compound A | 8.91 |
| Prazosin | 7.68 |

### Example 36

### Effects on urethral contractions (induced by noradrenaline injection and hypogastric-nerve stimulation) and on blood pressure in dogs after i.v. administration

The experiments were performed according to the method of Imagawa J. et al., *J Pharmacol. Methods* 22, 103-111 (1989), with substantial modifications, as follows: adult male beagle dogs, weighing 8-10 kg, were anaesthetised with pentobarbital sodium (30 mg/kg i.v. and 2 mg/kg/h i.v.), intubated and spontaneously ventilated with room air. In order to monitor systemic blood pressure (BP), a polyethylene (PE) catheter was introduced into the aortic arch through the left femoral artery. A collateral of the left femoral vein was cannulated for infusion of anaesthetic, and the right femoral vein was cannulated for administration of the compounds. For intraarterial (i.a.) injection of noradrenaline (NA), a PE catheter was introduced into the lower portion of the abdominal aorta via the right external iliac artery. Through such procedure, NA was selectively distributed to the lower urinary tract. A paramedian vertical suprapubic incision extending from the base of the pelvis to the mid-abdominal region was made and the bladder and the prostate were exposed. The bladder was manually emptied with a syringe. The hypogastric nerve was freed from surrounding tissue and cut 1 cm distal from the inferior mesenteric ganglion. The distal end of the right or left branch of the nerve was placed on a bipolar platinum electrode. Prostatic urethral pressure was monitored with a Mikro-tip catheter (5F) introduced into the bladder via the external urethral meatus, and withdrawn until the pressure transducer was positioned in the prostatic region of the urethra. A ligature was secured between the neck of the bladder and urethra to isolate the response of the latter and to avoid any interaction with the bladder. Another ligature was put around the Mikro-tip catheter at the external meatus, to secure the catheter itself. The hypogastric nerve stimulation was made with a train of rectangular pulses of 10-15 V, 10-30 Hz, width 5 msec, 8 sec duration.

After a stabilising period following the surgical procedure (30 minutes), in which arterial and prostatic urethral pressure were continuously monitored as basal values, i.a. administration of NA and stimulation of the hypogastric nerve were alternately made at intervals of 10 minutes.

The dose of NA and the parameter of hypogastric nerve stimulation chosen were such as to produce an increase of at least 100% in urethral pressure. The test compounds were administered i.v. in a cumulative manner with intervals of 15-20 min between administrations. Both i.a. injections of NA and stimulations of the hypogastric nerve were repeated 5 minutes after every dosing of test compound with intervals of about 5 minutes between two stimulations. In order to compare the effects of the administered compound, dose/response curves were constructed by computing, at the peak effect, the percent decrease in diastolic blood pressure and the percent inhibition of the increase in urethral pressure induced by both types of stimulation used. Linear regression equations were then used in order to evaluate the theoretical effectiveness as ED₂₅ (the effective dose inducing a 25% decrease in diastolic blood pressure) and ID₅₀ (the dose inhibiting by 50% the increase in urethral pressure).

### RESULTS

The effects obtained after i.v. administration of the compounds of Examples 1, 3 and 5 are shown in Table 3. The effects obtained after injection of prazosin and Compound A are also shown in the table.

**TABLE 3**

| *Data represent the active doses (expressed in µg*/*kg) inhibiting by 50% the urethral contractions (UP) induced by noradrenaline (NA) or by hypogastric-nerve stimulation (HYP), the active doses (expressed in µg*/*kg) in lowering diastolic blood pressure (DBP) and the ratios (DBP*/*UP) between the active doses (R1 and R2).* | | | | | | |
|---|---|---|---|---|---|---|
| **Example** | **UP ID**_{**50**} **NA** | **UP ID**_{**50**} **HYP** | **DBP ED**_{**25**} | | **R1 (DBP/NA)** | **R2 (DBP/HYP)** |
| 1 | 3.4 | | 345.4 | | 101.6 | |
| 3 | 1.3 | 2.4 | 514.7 | | 396 | 214 |
| 5 | 2.7 | 3.4 | 197.0 | | 73 | 58 |
| 6 | 2.4 | 4.5 | 196.7 | | 82 | 44 |
| Prazosin* | 3.6 | 10 | 6.6 | 10 | 1.83 | 1 |
| Compound A* | 2.4 | 6.7 | 243 | 177 | 101.2 | 26.4 |

| | | | | | | |
|---|---|---|---|---|---|---|
| * Data from Leonardi A. et al., *J. Pharmacol. Exp. Ther*. 281, 1272-1283 (1997). | | | | | | |

The pharmacological results confirm that the compounds of the invention are α₁-adrenoceptor antagonists with good selectivity for the α₁ₐ subtype, in particular with respect to the 5-HT_{1A} receptor, and good affinity also for the α_{1L} subtype, as far as in vitro data are concerned.

The in vivo pharmacological results confirm the extremely high uroselectivity of the compounds of the invention and justify their possible use in the treatment of obstructive diseases of the lower urinary tract, including BPH.

### Effective Amounts

The following represent guidelines to effective oral, parenteral or intravenous dose ranges expressed in mg/kg of body weight per day, for use in obstructive disorders of the lower urinary tract:

| | |
|---|---|
| General | 0.001-20 |
| Preferred | 0.05-3 |
| Most preferred | 0.5-2 |

The most preferred values refers to oral dosing. Intravenous dosages should be 10 to 100 fold lower. Selective-use dosages, i.e. dosages that are active in the lower urinary tract without any substantial effect on blood pressure, depend on the particular compound employed. Generally, in the case of a compound selective in inhibiting urethral contraction, up to four times the amount of the ED₅₀ used in inhibiting urethral contraction can be administered without any substantial effect on blood pressure. Further refinements and optimisation of dosages are possible using simple routine experiments. The active compounds of the invention may be orally administered, for example, with an inert diluent or with an edible carrier, or they may be enclosed in gelatine capsules, or they may be compressed into tablets. For the purpose of oral therapeutic administration, the active compounds of the invention may be incorporated with excipients and used in the form of tablets, troches, capsules, elixirs, suspensions, syrups, wafers, chewing gum and the like. These preparations should contain at least 0.5% of active compounds, but the amount of active ingredient may be varied depending upon the particular form and may conveniently be between 5% and about 70% of the weight of the unit. The amount of active compound in such compositions is such that a suitable dosage will be obtained although the desired dosage can be obtained by administering a plurality of dosage forms. The preferred compositions and preparations according to the invention are prepared so that an oral dosage unit form contains between 1.0 and 300 milligrams of active compound. The tablets, pills, capsules, troches and the like may also contain, for example, the following ingredients: a binder such as microcrystalline cellulose, gum tragacanth or gelatine; an excipient such as starch or lactose; a disintegrating agent such as alginic acid, sodium starch glycolate, cornstarch and the like; a lubricant such as magnesium stearate or hydrogenated castor oil; a glidant such as colloidal silicon dioxide; a sweetening agent such as sucrose or saccharin; and a flavouring agent such as peppermint, methyl salicylate or orange flavouring. When the dosage unit form is a capsule, it may contain, in addition to materials of the above type, a liquid carrier such as a fatty oil. Other dosage unit forms may contain other various materials which modify the physical form of the dosage unit, for example, as coatings. Thus, tablets or pills may be coated with sugar, shellac or other enteric coating agents. A syrup may contain, in addition to the active compounds, sucrose as a sweetening agent and certain preservatives, dyes, colouring and flavours. The materials used in preparing these various compositions should be pharmaceutically pure and nontoxic in the amounts used. For the purpose of parenteral therapeutic administration, the active compounds of the invention may be incorporated into a solution or suspension. These preparations should contain at least 0.1% of active compound, but this may be varied between 0.5 and about 30% of the weight thereof. The amount of active compound in such compositions is such that a suitable dosage will be obtained. The preferred compositions and preparations according to the inventions are prepared so that a parenteral dosage unit contains between 0.2 and 100 milligrams of active compound. The solutions or suspensions may also include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulphite; chelating agents such as ethylenediaminetetraacetic acid; buffers such as acetates; citrates or phosphates and agents for the adjustment of tonicity such as sodium chloride or dextrose. The parenteral multiple-dose vials may be of glass or plastics material.

Additional compositions suitable for administration by various routes and containing compounds according to the invention are also within the scope of the invention. Dosage forms, additional ingredients and routes of administration contemplated herein include those disclosed in US 4089969 and 5091182.

## Claims

1. A compound having the general formula I 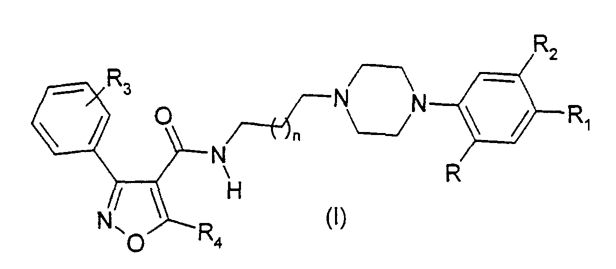 wherein
R represents an alkyl, alkoxy, polyfluoroalkoxy, hydroxy or trifluoromethanesulphonyloxy group;
each of R₁ and R₂ independently represents a hydrogen or halogen atom or a polyfluoroalkoxy or alkoxy group;
R₃ represents one or more substituents selected from hydrogen and halogen atoms and alkyl, alkoxy, nitro, amino, acylamino, cyano, alkoxycarbonyl and carboxamido groups;
R₄ represents a hydrogen atom or an alkyl or aralkyl group; and
n is 0, 1 or 2;
or an N-oxide or a pharmaceutically acceptable salt of such a compound.

2. A compound according to claim 1, wherein R represents a methyl, methoxy, 2,2,2-trifluoroethoxy, hydroxy or trifluoromethanesulphonyloxy group.

3. A compound according to claim 1 or claim 2, wherein R₁ represents a hydrogen, fluorine or chlorine atom.

4. A compound according to any preceding claim, wherein R₂ represents a hydrogen, chlorine or fluorine atom or a 2,2,2-trifluoroethoxy group.

5. A compound according to any preceding claim, wherein R₃ represents a hydrogen atom, a fluorine atom in the 2-, 3- or 4-position, a chlorine atom in the 2-position, a methoxy group in the 4-position, or a chlorine atom in the 2-position in combination with a chlorine or fluorine atom in the 6-position.

6. A compound according to any preceding claim, wherein R₄ represents a hydrogen atom or a methyl, ethyl, 2-phenylethyl or 3-phenylpropyl group.

7. A compound according to any preceding claim, wherein n is 1.

8. Any one of the following compounds:
• N-{3-[4-(5-Chloro-2-methoxyphenyl)-1-piperazinyl]-propyl}-5-methyl-3-phenylisoxazole-4-carboxamide,
• N-{3-[4-(5-Chloro-2-hydroxyphenyl)-1-piperazinyl]-propyl}-5-methyl-3-phenylisoxazole-4-carboxamide,
• 5-Methyl-3-phenyl-N-{3-[4-[2-(2,2,2-trifluoroethoxy)-phenyl]-1-piperazinyl]-propyl}-isoxazole-4-carboxamide,
• N-{3-[4-[2-Methoxy-5-(2,2,2-trifluoroethoxy)-phenyl]-1-piperazinyl]-propyl}-5-methyl-3-phenylisoxazole-4-carboxamide,
• N-{3-[4-[5-Fluoro-2-(2,2,2-trifluoroethoxy)-phenyl]-1-piperazinyl]-propyl}-5-methyl-3-phenylisoxazole-4-carboxamide,
• N-{3-[4-[4-Fluoro-2-(2,2,2-trifluoroethoxy)-phenyl]-1-piperazinyl]-propyl}-5-methyl-3-phenylisoxazole-4-carboxamide,
• N-{3-[4-[(5-Chloro-2-trifluoromethanesulphonyloxy)-phenyl]-1-piperazinyl]-propyl}-5-methyl-3-phenylisoxazole-4-carboxamide,
• 3-(4-Fluorophenyl)-N-{3-[4-[4-fluoro-2-(2,2,2-trifluoroethoxy)-phenyl]-1-piperazinyl]-propyl}-isoxazole-4-carboxamide,
• 3-(2-Chloro-6-fluorophenyl)-N-{3-[4-[4-fluoro-2-(2,2,2-trifluoroethoxy)-phenyl]-1-piperazinyl]-propyl}-5-methylisoxazole-4-carboxamide,
• 3-(2,6-Dichlorophenyl)-N-{3-[4-[4-fluoro-2-(2,2,2-trifluoroethoxy)-phenyl]-1-piperazinyl]-propyl}-5-methylisoxazole-4-carboxamide,
• 3-(2-Chlorophenyl)-N-{3-[4-[4-fluoro-2-(2,2,2-trifluoroethoxy)-phenyl]-1-piperazinyl]-propyl}-5-methylisoxazole-4-carboxamide,
• 3-(2-Chloro-6-fluorophenyl)-5-methyl-N-{3-[4-[2-(2,2,2-trifluoroethoxy)-phenyl]-1-piperazinyl]-propyl}-isoxazole-4-carboxamide,
• 3-(2-Chlorophenyl)-5-methyl-N-{3-[4-[2-(2,2,2-trifluoroethoxy)-phenyl]-1-piperazinyl]-propyl}-isoxazole-4-carboxamide,
• 3-(2,6-Dichlorophenyl)-5-methyl-N-{3-[4-[2-(2,2,2-trifluoroethoxy)-phenyl]-1-piperazinyl]-propyl}-isoxazole-4-carboxamide,
• N-{3-[4-[4-Fluoro-2-(2,2,2-trifluoroethoxy)-phenyl)-1-piperazinyl]-propyl}-3-phenylisoxazole-4-carboxamide,
• 3-(4-Fluorophenyl)-N-{3-[4-[2-(2,2,2-trifluoroethoxy)-phenyl]-1-piperazinyl]-propyl}-isoxazole-4-carboxamide,
• 3-Phenyl-N-{3-[4-[2-(2,2,2-trifluoroethoxy)-phenyl]-1-piperazinyl]-propyl}-isoxazole-4-carboxamide,
• 3-(4-Fluorophenyl)-N-{3-[4-[4-fluoro-2-(2,2,2-trifluoroethoxy)-phenyl]-1-piperazinyl]-propyl}-5-methylisoxazole-4-carboxamide,
• 5-Ethyl-3-phenyl-N-{3-[4-[2-(2,2,2-trifluoroethoxy)-phenyl]-1-piperazinyl]-propyl}-isoxazole-4-carboxamide,
• 5-Ethyl-N-{3-[4-[4-fluoro-2-(2,2,2-trifluoroethoxy)-phenyl]-1-piperazinyl]-propyl}-3-phenylisoxazole-4-carboxamide,
• 3-(4-Fluorophenyl)-5-methyl-N-{3-[4-[2-(2,2,2-trifluoroethoxy)-phenyl]-1-piperazinyl]-propyl}-isoxazole-4-carboxamide,
• 3-(2-Fluorophenyl)-N-{3-[4-[4-fluoro-2-(2,2,2-trifluoroethoxy)-phenyl]-1-piperazinyl]-propyl}-5-methylisoxazole-4-carboxamide,
• 3-(2-Fluorophenyl)-5-methyl-N-{3-[4-[2-(2,2,2-trifluoroethoxy)-phenyl]-1-piperazinyl]-propyl}-isoxazole-4-carboxamide,
• 3-(3-Fluorophenyl)-N-{3-[4-[4-fluoro-2-(2,2,2-trifluoroethoxy)-phenyl]-1-piperazinyl]-propyl}-5-methylisoxazole-4-carboxamide,
• 3-(3-Fluorophenyl)-5-methyl-N-{3-[4-[2-(2,2,2-trifluoroethoxy)-phenyl]-1-piperazinyl]-propyl}-isoxazole-4-carboxamide,
• 3-(3-Fluorophenyl)-5-methyl-N-{3-[4-[2-(2,2,2-trifluoroethoxy)-phenyl]-1-piperazinyl]-propyl}-isoxazole-4-carboxamide,
• N-{3-[4-[4-Fluoro-2-(2,2,2-trifluoroethoxy)-phenyl]-1-piperazinyl]-propyl}-3-(4-methoxyphenyl)-5-methylisoxazole-4-carboxamide,
• N-{3-[4-[4-Fluoro-2-(2,2,2-trifluoroethoxy)-phenyl]-1-piperazinyl]-propyl}-3-phenyl-5-(2-phenylethyl)-isoxazole-4-carboxamide,
• N-{3-[4-[4-Fluoro-2-(2,2,2-trifluoroethoxy)-phenyl]-1-piperazinyl]-propyl}-3-phenyl-5-(3-phenylpropyl)-isoxazole-4-carboxamide,
• N-{3-[4-(4-Fluoro-2-methoxyphenyl)-1-piperazinyl]-propyl}-5-methyl-3-phenylisoxazole-4-carboxamide,
• N-{3-[4-(4-Fluoro-2-methylphenyl)-1-piperazinyl]-propyl}-5-methyl-3-phenylisoxazole-4-carboxamide,
• N-{3-[4-(4-Chloro-2-methylphenyl)-1-piperazinyl]-propyl}-5-methyl-3-phenylisoxazole-4-carboxamide, and
• N-{3-[4-[4-Fluoro-2-(1-methylethoxy)-phenyl]-1-piperazinyl]-propyl}-5-methyl-3-phenylisoxazole-4-carboxamide,
or an N-oxide or a pharmaceutically acceptable salt of such a compound.

9. A pharmaceutical composition comprising a compound according to any preceding claim, or an N-oxide or a pharmaceutically acceptable salt of such a compound, in admixture with a pharmaceutically acceptable diluent or carrier.

10. A method for the preparation of a compound having the general formula I 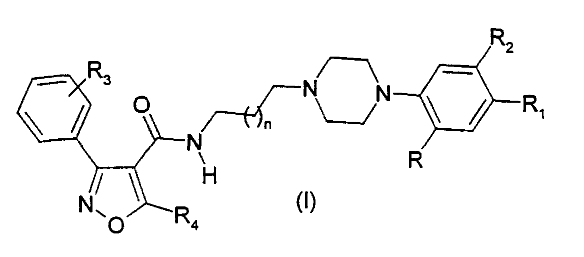 wherein
R represents an alkyl, alkoxy, polyfluoroalkoxy, hydroxy or trifluoromethanesulphonyloxy group;
each of R₁ and R₂ independently represents a hydrogen or halogen atom or a polyfluoroalkoxy or alkoxy group;
R₃ represents one or more substituents selected from hydrogen and halogen atoms and alkyl, alkoxy, nitro, amino, acylamino, cyano, alkoxycarbonyl and carboxamido groups;
R₄ represents a hydrogen atom or an alkyl or aralkyl group; and
n is 0, 1 or 2;
the method comprising condensing a 4-carboxy-isoxazole derivative of the general formula **1**, or an ester, amide or anhydride thereof, 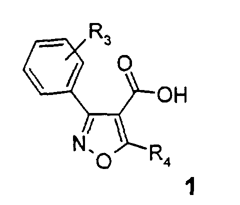 wherein R₃ and R₄ are as above defined with an N-(ω-aminoalkyl)-N'-phenylpiperazine derivative of the general formula **2** 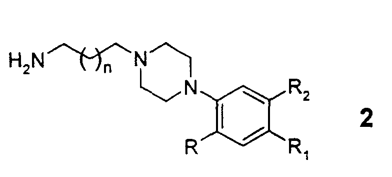 wherein n, R, R₁, and R₂ are as above defined.

11. A method according to claim 10, wherein the condensation is carried out in the presence of a condensing agent such as dicyclohexylcarbodiimide or diethyl cyanophosphonate, optionally in the presence of a promoting agent such as N-hydroxysuccinimide or 4-dimethylaminopyridine or N,N'-carbonyldiimidazole, in an aprotic solvent or in a chlorinated solvent at a temperature of from -10 to 140°C.

12. A method for the preparation of a compound having the general formula I 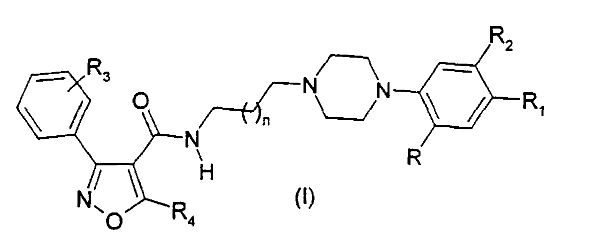 wherein
R represents an alkyl, alkoxy, polyfluoroalkoxy, hydroxy or trifluoromethanesulphonyloxy group;
each of R₁ and R₂ independently represents a hydrogen or halogen atom or a polyfluoroalkoxy or alkoxy group;
R₃ represents one or more substituents selected from hydrogen and halogen atoms and alkyl, alkoxy, nitro, amino, acylamino, cyano, alkoxycarbonyl and carboxamido groups;
R₄ represents a hydrogen atom or an alkyl or aralkyl group; and
n is 0, 1 or 2;
the method comprising condensing a 4-carboxy-isoxazole derivative of the general formula **1**, or an ester, amide or anhydride thereof, 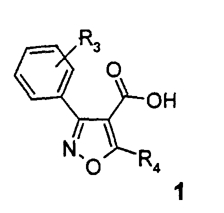 wherein R₃ and R₄ are as above defined with an amine of the general formula H₂NCH₂(CH₂)ₙCH₂X wherein X represents a halogen atom or a hydroxy group and n is as above defined; in the resultant compound of the general formula **3** 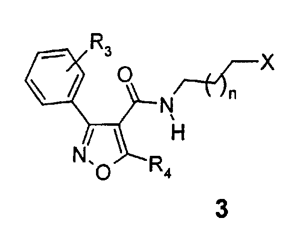 and condensing the compound of the general formula 3, when X represents a hydroxy group, converting it to a leaving group, such as an alkylsulphonyloxy or arylsulphonyloxy group, with a phenylpiperazine derivative 4 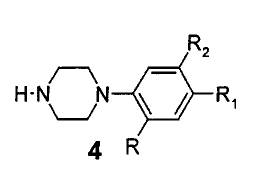 wherein R, R₁ and R₂ are as above defined.

13. A method according to claim 12, wherein the condensation of the 4-carboxy-isoxazole derivative **1** with the amine H₂NCH₂(CH₂)ₙCH₂X is carried out in the presence of a condensing agent such as dicyclohexylcarbodiimide or diethyl cyanophosphonate, optionally in the presence of a promoting agent such as N-hydroxysuccinimide or 4-dimethylaminopyridine or N,N'-carbonyldiimidazole, in an aprotic solvent or in a chlorinated solvent at a temperature of from 10 to 140°C.

14. A method according to claim 12 or claim 13, wherein the condensation of the compound **3** with the phenylpiperazine derivative **4** is carried out without solvent, or alternatively in a polar solvent such as dimethylformamide or acetonitrile or methanol, at a temperature of from 20 to 200°C, preferably in the presence of a base such as potassium carbonate.

## Patentansprüche

1. Verbindung mit der allgemeinen Formel I 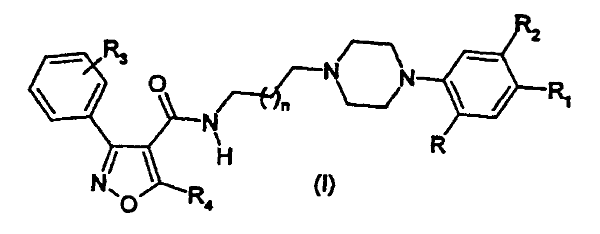 wobei
R eine Alkyl-, Alkoxy-, Polyfluoralkoxy-, Hydroxy- oder Trifluormethansulfonyloxy-Gruppe darstellt,
R₁ und R₂ jeweils unabhängig voneinander ein Wasserstoff- oder Halogenatom darstellen oder eine Polyfluoralkoxy- oder Alkoxy-Gruppe,
R₃ einen oder mehrere Substituenten darstellt, ausgewählt aus Wasserstoff und Halogenatomen, sowie Alkyl-, Alkoxy-, Nitro-, Amino-Acylamino-, Cyano-, Alkoxycarbonyl- und Carboxamido-Gruppen,
R₄ ein Wasserstoffatom oder eine Alkyl- oder Aralkyl-Gruppe darstellt, und
n 0,1 oder 2 ist,
oder ein N-Oxid oder ein pharmazeutisch akzeptables Salz einer solchen Verbindung.

2. Verbindung gemäß Anspruch 1, wobei R eine Methyl-, Methoxy-, 2,2,2-Trifluorethoxy-, Hydroxy- oder Trifluormethansulfonyloxy-Gruppe ist.

3. Verbindung gemäß Anspruch 1 oder 2, wobei R₁ ein Wasserstoff-, Fluoroder Chloratom darstellt.

4. Verbindung gemäß einem der vorhergehenden Ansprüche, wobei R₂ ein Wasserstoff-, Chlor- oder Fluoratom oder eine 2,2,2-Trifluorethoxy-Gruppe ist.

5. Verbindung gemäß einem der vorhergehenden Ansprüche, wobei R₃ ein Wasserstoffatom, ein Fluoratom in 2-, 3- oder 4-Position, ein Chloratom in der 2-Position, eine Methoxy-Gruppe in der 4-Position oder ein Chloratom in der 2-Position in Verbindung mit einem Chlor- oder Fluoratom in der 6-Position ist.

6. Verbindung gemäß einem der vorhergehenden Ansprüche, wobei R₄ ein Wasserstoffatom oder eine Methyl-, Ethyl-, 2-Phenylethyl- oder 3-Phenylpropyl-Gruppe darstellt.

7. Verbindung gemäß einem der vorhergehenden Ansprüche, wobei n 1 ist.

8. Eine der folgenden Verbindungen:
• N-{3-[4-(5-Chlor-2-methoxyphenyl)-1-piperazinyl]propyl}-5-methyl-3-phenylisoxazol-4-carboxamid,
• N-{3-[4-(5-Chlor-2-hydroxyphenyl)-1-piperazinyl]propyl}-5-methyl-3-phenylisoxazol-4-carboxamid,
• 5-Methyl-3-phenyl-N-{3-[4-[2-(2,2,2-trifluorethoxy)phenyl]-1-piperazinyl]propyl}-isoxazol-4-carboxamid,
• N-{3-[4-[2-Methoxy-5-(2,2,2-trifluorethoxy)phenyl]-1-piperazinyl]propyl}-5-methyl-3-phenylisoxazol-4-carboxamid,
• N-{3-[4-[5-Fluor-2-(2,2,2-trifluorethoxy)phenyl}-1-
• N-{3-[4-[4-Fluor-2-(2,2,2-trifluorethoxy)phenyl]-1-piperazinyl]propyl)-5-methyl-3-phenylisoxazol-4-carboxamid,
• N-{3-[4-[(5-Chlor-2-trifluormethansulfonyloxy)phenyl]-1-piperazinyl]propyl}-5-methyl-3-phenylisoxazol-4-carboxamid,
• 3-(4-Fluorphenyl)-N-{3-[4-[4-fluor-2-(2,2,2-trifluorethoxy)phenyl]-1-piperazinyl]propyl}isoxazol-4-carboxamid,
• 3-(2-Chlor-6-fluorphenyl)-N-{3-[4-[4-fluor-2-(2,2,2-trifluorethoxy) phenyl]-1-piperazinyl]propyl}-5-methylisoxazol-4-carboxamid,
• 3-(2,6-Dichlorphenyl)-N-{3-[4-[4-fluor-2-(2,2,2-trifluorethoxy) phenyl]-1-piperazinyl]propyl}-5-methylisoxazol-4-carboxamid,
• 3-(2-Chlorphenyl)-N-{3-[4-[4-fluor-2-(2,2,2-trifluorethoxy)phenyl]-1-piperazinyl]propyl}-5-methylisoxazol-4-carboxamid,
• 3-(2-Chlor-6-fluorphenyl)-5-methyl-N-{3-[4-(2-(2,2,2-trifluorethoxy) phenyl]-1-piperazinyl]propyl}isoxazol-4-carboxamid,
• 3-(2-Chlorphenyl)-5-methyl-N-(3-[4-[2-(2,2,2-trifluorethoxy)phenyl]-1-piperazinyl]propyl}isoxazol-4-carboxamid,
• 3-(2,6-Dichlorphenyl)-5-methyl-N-{3-[4-[2-(2,2,2-trifluorethoxy) phenyl]-1-piperazinyl]propyl}isoxazol-4-carboxamid,
• N-{3-[4-[4-Fluor-2-(2,2.2-trifluorethoxy)phenyl]-1-piperazinyl]propyl}-3-phenylisoxazol-4-carboxamid,
• 3-(4-Fluorphenyl)-N-{3-[4-[2-(2,2,2-trifluorethoxy)phenyl]-1-piperazinyl]propyl}isoxazol-4-carboxamid,
• 3-Phenyl-N-{3-[4-(2-(2.2.2-trifluorethoxy)phenyl]-1-piperazinyl]propyl}isoxazol-4-carboxamid
• 3-(4-Fluorphenyl)-N-{3-[4-[4-fluor-2-(2,2,2-trifluorethoxy)phenyl]-1-piperazinyl]propyl}-5-methylisoxazol-4-carboxamid,
• 5-Ethyl-3-phenyl-N-{3-[4-[2-(2,2,2-trifluorethoxy)phenyl]-1-piperazinyl]propyl}isoxazol-4-carboxamid,
• 5-Ethyl-N-{3-[4-[4-fluor-2-(2,2,2-trifluorethoxy)phenyl]-1-piperazinyl]propyl}-3-phenylisoxazol-4-carboxamid,
• 3-(4-Fluorphenyl)-5-methyl-N-{3-[4-[2-(2,2,2-trifluorethoxy)phenyl]-1-piperazinyl]propyl}isoxazol-4-carboxamid,
• 3 -(2-Fluorphenyl)-N-{3-[4-[4-fluor-2-(2,2,2-trifluorethoxy)phenyl]-1-
• 3-(2-Fluorphenyl)-N-{3-[4-[4-fluor-2-(2,2,2-trifluorethoxy)phenyl]-1-piperazinyl]propyl}-5-methylisoxazol-4-carboxamid,
• 3-(2-Fluorphenyl)-5-methyl-N-{3-[4-[2-(2,2,2-trifluorethoxy)phenyl]-1-piperazinyl]propyl}isoxazol-4-carboxamid,
• 3-(3-Fluorphenyl)-N-(3-[4-[4-fluor-2-(2,2,2-trifluorethoxy)phenyl]-1-piperazinyl]propyl}-5-methylisoxazol-4-carboxamid,
• 3-(3-Fluorphenyl)-5-methyl-N-{3-[4-[2-(2,2,2-trifluorethoxy)phenyl]-1-piperazinyl]propyl}isoxazol-4-carboxamid,
• 3-(3-Fluorphenyl)-5-methyl-N-{3-[4-{2-(2,2,2-trifluoroethoxy) phenyl]-1-piperazinyl]propyl}isoxazol-4-carboxamid,
• N-{3-[4-[4-Fluor-2-(2,2,2-trifluorethoxy)phenyl]-1-piperazinyl] propyl}-3-(4-methoxyphenyl)-5-methylisoxazol-4-carboxamid,
• N-{3-[4-[4-Fluor-2-(2,2,2-trifluorethoxy)phenyl]-1-piperazinyl] propyl}-3-phenyl-5-(2-phenylethyl)isoxazol-4-carboxamid,
• N-{3-[4-[4-Fluor-2-(2,2,2-trifluorethoxy)phenyl]-1-piperazinyl] propyl}-3-phenyl-5-(3-phenylpropyl)isoxazol-4-carboxamid,
• N-{3-[4-(4-Fluor-2-methoxyphenyl)-1-piperazinyl]propyl}-5-methyl-3-phenylisoxazol-4-carboxamid,
• N-{3-[4-(4-Fluor-2-methylphenyl)-1-piperazinyl]propyl}-5-methyl-3-phenylisoxazol-4-carboxamid,
• N-{3-[4-(4-Chlor-2-methylphenyl)-1-piperazinyl]propyl}-5-methyl-3-phenylisoxazol-4-carboxamid, und
• N-{3-[4-[4-Fluor-2-(1-methylethoxy)phenyl]-1-piperazinyl]propyl}-5-methyl-3-phenylisoxazol-4-carboxamid
oder ein N-Oxid oder ein pharmazeutisch akzeptables Salz einer solchen Verbindung.

9. Eine pharmazeutische Zusammensetzung aus einer Verbindung gemäß einem der vorhergehenden Ansprüche oder einem N-Oxid oder einem pharmazeutisch akzeptablen Salz einer solchen Verbindung, vermischt mit einem pharmazeutisch akzeptablen Verdünnungsmittel oder Träger.

10. Verfahren für die Herstellung einer Verbindung mit der allgemeinen Formel I 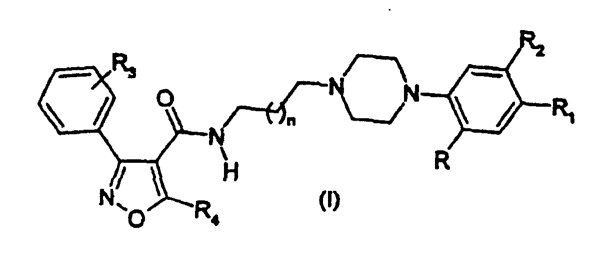 wobei
R eine Alkyl-, Alkoxy-, Polyfluoralkoxy-, Hydroxy- oder Trifluormethansulfonyloxy-Gruppe ist,
R₁ und R₂ unabhängig voneinander jeweils ein Wasserstoff- oder Halogenatom oder eine Polyfluoralkoxy- oder Alkoxy-Gruppe darstellen,
R₃ einen oder mehrere Substituenten darstellt, ausgewählt aus Wasserstoff- und Halogenatomen, sowie Alkyl-, Alkoxy-, Nitro-, Amino-, Acylamino-, Cyano-, Alkoxycarbonyl- und Carboxamido-Gruppen,
R₄ ein Wasserstoffatom oder eine Alkyl- oder Aralkyl-Gruppe darstellt, sowie
n 0,1 oder 2 ist,
wobei das Verfahren aufweist: Kondensieren eines 4-Carboxyisoxazol-Derivates der allgemeinen Formel 1, oder einen Ester oder ein Amid oder ein Anhydrid davon, 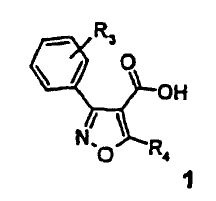 wobei R₃ und R₄ wie oben definiert sind, mit einem N-(ω-Aminoalkyl)-N'-phenylpiperazin-Derivat der allgemeinen Formel 2 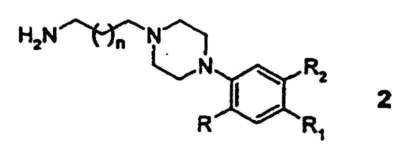 wobei n, R, R₁ und R₂ wie oben definiert sind.

11. Verfahren gemäß Anspruch 10, wobei die Kondensation in Gegenwart eines Kondensationshilfsmittels wie z. B. die Dicyclohexylcarbodiimid oder Diethylcyanophosphonat ausgeführt wird, wahlweise in Gegenwart eines Beschleunigungsmittels wie z. B. N-Hydroxysuccinimid oder 4-Dimethylaminopyridin oder N,N'-Carbonyldiimidazol in einem aprotischen Lösungsmittel oder in einem chlorierten Lösungsmittel bei einer Temperatur von -10 bis 140° C.

12. Verfahren für die Herstellung einer Verbindung mit der allgemeinen Formel I 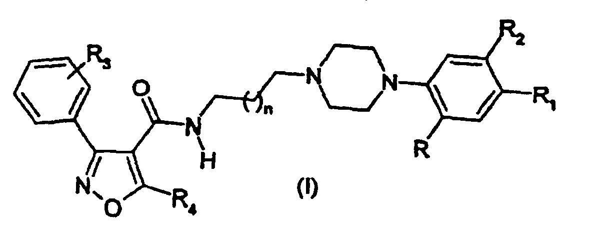 wobei
R eine Alkyl-, Alkoxy-, Polyfluoralkoxy-, Hydroxy- oder Trifluormethansulfonyloxy-Gruppe ist,
R₁ und R₂ unabhängig voneinander jeweils ein Wasserstoff- oder Halogenatom oder eine Polyfluoralkoxy- oder Alkoxy-Gruppe darstellen,
R₃ einen oder mehrere Substituenten darstellt, ausgewählt aus Wasserstoffund Halogenatomen, sowie Alkyl-, Alkoxy-, Nitro-, Amino-, Acylamino-, Cyano-, Alkoxycarbonyl- und Carboxamido-Gruppen,
R₄ ein Wasserstoffatom oder eine Alkyl- oder Aralkyl-Gruppe darstellt, und
n 0,1 oder 2 ist,
wobei das Verfahren aufweist: Kondensieren eines 4-Carboxyisoxazol-Derivates der allgemeinen Formel 1 oder eines Esters, Amids oder Anhydrids davon, 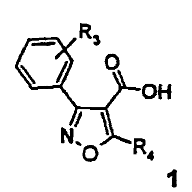 wobei R₃ und R₄ wie oben beschrieben sind, mit einem Amin der allgemeinen Formel H₂NCH₂(CH₂)ₙCH₂X, wobei X ein Halogenatom oder eine Hydroxy-Gruppe darstellt und n wie oben definiert ist, zur daraus resultierenden Verbindung der allgemeinen Formel 3 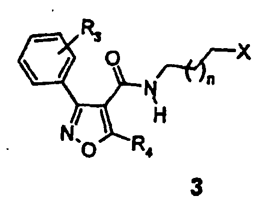 und Kondensieren der Verbindung der allgemeinen Formel 3, wenn X eine Hydroxy-Gruppe darstellt konvertieren von dieser in eine Abgangsgruppe wie z. B. eine Alkylsulfonyloxy- oder Arylsulfonyloxy-Gruppe, mit einem Phenylpiperazin-Derivat 4 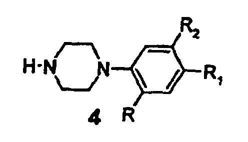 wobei R, R₁ und R₂ wie oben definiert sind.

13. Verfahren gemäß Anspruch 12, wobei die Kondensation des 4-Carboxyisoxazol-Derivates 1 mit dem Amin H₂NCH₂(CH₂)ₙCH₂X in Gegenwart eines Kondensationshilfsmittels wie z. B. Dicyclohexylcarbodiimid oder Diethylcyanophosphonat durchgeführt wird, wahlweise in Gegenwart eines Beschleunigungsmittels wie z. B. N-Hydroxysuccinimid oder 4-Dimethylaminopyridin oder N,N'-Carbonyldiimidazol, in einem aprotischen Lösungsmittel oder in einem chlorierten Lösungsmittel bei einer Temperatur von 10 bis 140° C.

14. Verfahren gemäß Anspruch 12 oder 13, wobei die Kondensation der Verbindung 3 mit dem Phenylpiperazin-Derivat 4 ohne Lösungsmittel ausgeführt wird oder alternativ in einem polaren Lösungsmittel, wie z. B. Dimethylformamid oder Acetontril oder Methanol ausgeführt wird, bei einer Temperatur von 20 bis 200° C, vorzugsweise in Gegenwart einer Base wie z. B. Kaliumcarbonat.

## Revendications

1. Composé ayant la formule générale I 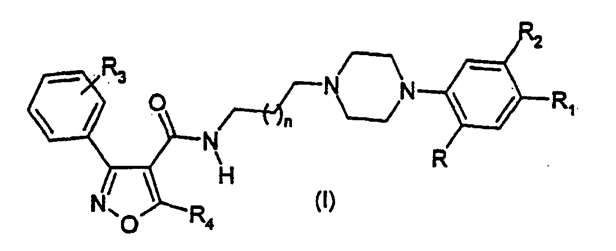 dans laquelle
R représente un groupe alkyle, alcoxy, polyfluoroalcoxy, hydroxy ou trifluorométhanesulfonyloxy ;
chacun de R₁ et R₂ représente indépendamment un atome d'hydrogène ou d'halogène ou un groupe polyfluoroalcoxy ou alcoxy ;
R₃ représente un ou plusieurs substituants choisis parmi des atomes d'hydrogène et d'halogène et des groupes alkyle, alcoxy, nitro, amino, acylamino, cyano, alcoxycarbonyle et carboxamido ;
R₄ représente un atome d'hydrogène ou un groupe alkyle ou aralkyle ; et
n est 0, 1 ou 2 ;
ou un N-oxyde ou un sel pharmaceutiquement acceptable d'un tel composé.

2. Composé selon la revendication 1, dans lequel R représente un groupe méthyle, méthoxy, 2,2,2-trifluoroéthoxy, hydroxy ou trifluorométhanesulfonyloxy.

3. Composé selon la revendication 1 ou la revendication 2, dans lequel R₁ représente un atome d'hydrogène, de fluor ou de chlore.

4. Composé selon l'une quelconque des revendications précédentes, dans lequel R₂ représente un atome d'hydrogène, de chlore ou de fluor ou un groupe 2,2,2-trifluoroéthoxy.

5. Composé selon l'une quelconque des revendications précédentes, dans lequel R₃ représente un atome d'hydrogène, un atome de fluor en position 2, 3 ou 4, un atome de chlore en position 2, un groupe méthoxy en position 4 ou un atome de chlore en position 2 en combinaison avec un atome de chlore ou de fluor en position 6.

6. Composé selon l'une quelconque des revendications précédentes, dans lequel R₄ représente un atome d'hydrogène ou un groupe méthyle, éthyle, 2-phényléthyle ou 3-phénylpropyle.

7. Composé selon l'une quelconque des revendications précédentes, dans lequel n est 1.

8. L'un quelconque des composés suivants :
N-{3-[4-(5-chloro-2-méthoxyphényl)-1-pipérazinyl]-propyl}-5-méthyl-3-phénylisoxazole-4-carboxamide,
N-{3-[4-(5-chloro-2-hydroxyphényl)-1-pipérazinyl]-propyl}-5-méthyl-3-phénylisoxazole-4-carboxamide,
5-méthyl-3-phényl-N-{3-[4-[2-(2,2,2-trifluoroéthoxy)-phényl]-1-pipérazinyl]-propyl}isoxazole-4-carboxamide,
N-{3-[4-[2-méthoxy-5-(2,2,2-trifluoroéthoxy)-phényl]-1-pipérazinyl]-propyl}-5-méthyl-3-phénylisoxazole-4-carboxamide,
N-{3-[4-[5-fluoro-2-(2,2,2-trifluoroéthoxy)-phényl]-1-pipérazinyl]-propyl}-5-méthyl-3-phénylisoxazole-4-carboxamide,
N-{3-[4-[4-fluoro-2-(2,2,2-trifluoroéthoxy)-phényl]-1-pipérazinyl]-propyl}-5-méthyl-3-phénylisoxazole-4-carboxamide,
N-{3-[4-[(5-chloro-2-trifluorométhanesulfonyloxy)-phényl]-1-pipérazinyl]-propyl}-5-méthyl-3-phénylisoxazole-4-carboxamide,
3-(4-fluorophényl)-N-{3-[4-[4-fluoro-2-(2,2,2-trifluoroéthoxy)-phényl]-1-pipérazinyl]-propyl}-isoxazole-4-carboxamide,
3-(2-chloro-6-fluorophényl)-N-{3-[4-[4-fluoro-2-(2,2,2-trifluoroéthoxy)-phényl]-1-pipérazinyl]-propyl}-5-méthylisoxazole-4-carboxamide,
3-(2,6-dichlorophényl)-N-{3-[4-[4-fluoro-2-(2,2,2-trifluoroéthoxy)-phényl]-1-pipérazinyl]-propyl}-5-méthylisoxazole-4-carboxamide,
3-(2-chlorophényl)-N-{3-[4-[4-fluoro-2-(2,2,2-trifluoroéthoxy)-phényl]-1-pipérazinyl]-propyl}-5-méthylisoxazole-4-carboxamide,
3-(2-chloro-6-fluorophényl)-5-méthyl-N-{3-[4-[2-(2,2,2-trifluoroéthoxy)-phényl]-1-pipérazinyl]-propyl}-isoxazole-4-carboxamide,
3-(2-chlorophényl)-5-méthyl-N-{3-[4-[2-(2,2,2-trifluoroéthoxy)-phényl]-1-pipérazinyl]-propyl}-isoxazole-4-carboxamide,
3-(2,6-dichlorophényl)-5-méthyl-N-{3-[4-[2-(2,2,2-trifluoroéthoxy)-phényl]-1-pipérazinyl]-propyl}-isoxazole-4-carboxamide,
N-{3-[4-[4-fluoro-2-(2,2,2-trifluoroéthoxy)-phényl]-1-pipérazinyl]-propyl}-3-phénylisoxazole-4-carboxamide,
3-(4-fluorophényl)-N-{3-[4-[2-(2,2,2-trifluoroéthoxy)-phényl]-1-pipérazinyl]-propyl}-isoxazole-4-carboxamide,
3-phényl-N-{3-[4-[2-(2,2,2-trifluoroéthoxy)-phényl]-1-pipérazinyl]-propyl}-isoxazole-4-carboxamide,
3-(4-fluorophényl)-N-{3-[4-[4-fluoro-2-(2,2,2-trifluoroéthoxy)-phényl]-1-pipérazinyl]-propyl}-5-méthylisoxazole-4-carboxamide,
5-éthyl-3-phényl-N-{3-[4-(2-(2,2,2-trifluoroéthoxy)-phényl]-1-pipérazinyl]-propyl}-isoxazole-4-carboxamide,
5-éthyl-N-{3-[4-[4-fluoro-2-(2,2,2-trifluoroéthoxy)-phényl]-1-pipérazinyl]-propyl}-3-phénylisoxazole-4-carboxamide,
3-(4-fluorophényl)-5-méthyl-N-{3-[4-[2-(2,2,2-trifluoroéthoxy)-phényl]-1-pipérazinyl]-propyl}-isoxazole-4-carboxamide,
3-(2-fluorophényl)-N-{3-[4-[4-fluoro-2-(2,2,2-trifluoroéthoxy)-phényl]-1-pipérazinyl]-propyl}-5-méthylisoxazole-4-carboxamide,
3-(2-fluorophényl)-5-méthyl-N-{3-[4-[2-(2,2,2-trifluoroéthoxy)-phényl]-1-pipérazinyl]-propyl}-isoxazole-4-carboxamide,
3-(3-fluorophényl)-N-{3-[4-[4-fluoro-2-(2,2,2-trifluoroéthoxy)-phényl]-1-pipérazinyl]-propyl}-5-méthylisoxazole-4-carboxamide,
3-(3-fluorophényl)-5-méthyl-N-{3-[4-[2-(2,2,2-trifluoroéthoxy)-phényl]-1-pipérazinyl]-propyl}-isoxazole-4-carboxamide,
3-(3-fluorophényl)-5-méthyl-N-{3-[4-[2-(2,2,2-trifluoroéthoxy)-phényl]-1-pipérazinyl]-propyl}-isoxazole-4-carboxamide,
N-{3-[4-[4-fluoro-2-(2,2,2-trifluoroéthoxy)-phényl]-1-pipérazinyl]-propyl}-3-(4-méthoxyphényl)-5-méthylisoxazole-4-carboxamide,
N-{3-[4-[4-fluoro-2-(2,2,2-trifluoroéthoxy)-phényl]-1-pipérazinyl]-propyl}-3-phényl-5-(2-phényléthyl)-isoxazole-4-carboxamide,
N-{3-[4-[4-fluoro-2-(2,2,2-trifluoroéthoxy)-phényl]-1-pipérazinyl]-propyl}-3-phényl-5-(3-phénylpropyl)-isoxazole-4-carboxamide,
N-{3-[4-(4-fluoro-2-méthoxyphényl)-1-pipérazinyl]-propyl}-5-méthyl-3-phénylisoxazole-4-carboxamide,
N-{3-[4-(4-fluoro-2-méthylphényl)-1-pipérazinyl]-propyl}-5-méthyl-3-phénylisoxazole-4-carboxamide,
N-{3-[4-(4-chloro-2-méthylphényl)-1-pipérazinyl]-propyl}-5-méthyl-3-phénylisoxazole-4-carboxamide, et
N-{3-[4-(4-fluoro-2-(1-méthyléthoxy)-1-pipérazinyl]-propyl}-5-méthyl-3-phénylisoxazole-4-carboxamide,
ou un N-oxyde ou un sel pharmaceutiquement acceptable d'un tel composé.

9. Composition pharmaceutique comprenant un composé selon l'une quelconque des revendications précédentes, ou un N-oxyde ou un sel pharmaceutiquement acceptable d'un tel composé, en mélange avec un diluant ou véhicule pharmaceutiquement acceptable.

10. Procédé pour la préparation d'un composé ayant la formule générale I 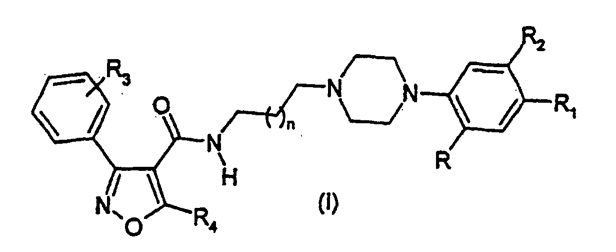 dans laquelle
R représente un groupe alkyle, alcoxy, polyfluoroalcoxy, hydroxy ou trifluorométhanesulfonyloxy;
chacun de R₁ et R₂ représente indépendamment un atome d'hydrogène ou d'halogène ou un groupe polyfluoroalcoxy ou alcoxy ;
R₃ représente un ou plusieurs substituants choisis parmi les atomes d'hydrogène et d'halogène et des groupes alkyle, alcoxy, nitro, amino, acylamino, cyano, alcoxycarbonyle et carboxamido ;
R₄ représente un atome d'hydrogène ou un groupe alkyle ou aralkyle ; et
n est 0, 1 ou 2 ;
le procédé comprenant la condensation d'un dérivé 4-carboxy-isoxazole de formule générale 1, ou un ester, un amide ou un anhydride de celui-ci, 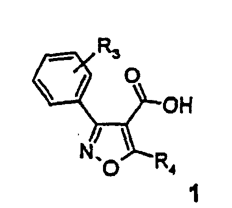 dans laquelle R₃ et R₄ sont tels que définis ci-dessus avec un dérivé N-(ω-aminoalkyl)-N'-phényl-pipérazine de formule générale 2 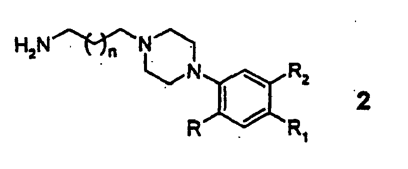 dans laquelle n, R, R₁ et R₂ sont tels que définis ci-dessus.

11. Procédé selon la revendication 10, dans lequel on réalise la condensation en présence d'un agent de condensation tel que le dicyclohexylcarbodiimide ou le cyanophosphonate de diéthyle, éventuellement en présence d'un agent activant tel qu'un N-hydroxysuccinimide ou une 4-diméthylaminopyridine ou un N,N'-carbonyldiimidazole, dans un solvant aprotique ou dans un solvant chloré à une température allant de -10 à 140 °C.

12. Procédé pour la préparation d'un composé ayant la formule générale I 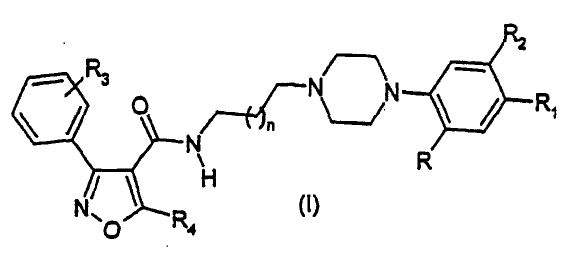 dans laquelle
R représente un groupe alkyle, alcoxy, polyfluoroalcoxy, hydroxy ou trifluorométhanesulfonyloxy ;
chacun de R₁ et R₂ représente indépendamment un atome d'hydrogène ou d'halogène ou un groupe polyfluoroalcoxy ou alcoxy ;
R₃ représente un ou plusieurs substituants choisis parmi les atomes d'hydrogène et d'halogène et les groupes alkyle, alcoxy, nitro, amino, acylamino, cyano, alcoxycarbonyle et carboxamido ;
R₄ représente un atome d'hydrogène ou un groupe alkyle ou aralkyle ; et
n est 0, 1 ou 2 ;
le procédé comprenant la condensation d'un dérivé 4-carboxy-isoxazole de formule générale 1, ou d'un ester, d'un amide ou d'un anhydride de celui-ci, 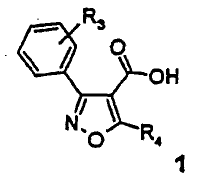 dans laquelle R₃ et R₄ sont tels que définis ci-dessus avec une amine de formule générale H₂NCH₂(CH₂)ₙCH₂X dans laquelle X représente un atome d'halogène ou un groupe hydroxy et n est tel que défini ci-dessus ; pour obtenir le composé résultant de formule générale 3 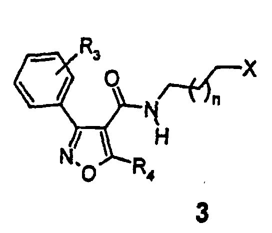 et la condensation du composé de formule générale 3, lorsque X représente un groupe hydroxy, en le transformant en un groupe partant, tel qu'un groupe alkylsulfonyloxy ou arylsulfonyloxy, avec un dérivé phénylpipérazine 4 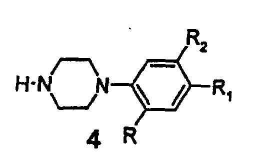 dans laquelle R, R₁ et R₂ sont tels que définis ci-dessus.

13. Procédé selon la revendication 12, dans lequel la condensation du dérivé 4-carboxy-isoxazole 1 avec l'amine H₂NCH₂(CH₂)ₙCH₂X est réalisée en présence d'un agent de condensation tel qu'un dicyclohexylcarbodiimide ou un diéthylcyanophosphonate, éventuellement en présence d'un agent activant tel qu'un N-hydroxysuccinimide ou une 4-diméthylaminopyridine ou un N,N'-carbonyldiimidazole, dans un solvant aprotique ou dans un solvant chloré à une température allant de 10 à 140°C.

14. Procédé selon la revendication 12 ou la revendication 13, dans lequel la condensation du composé 3 avec le dérivé phénylpipérazine 4 est réalisée sans solvant, ou en tant qu'alternative dans un solvant polaire tel que le diméthylformamide ou l'acétonitrile ou le méthanol, à une température allant de 20 à 200 °C, de préférence en présence d'une base telle que le carbonate de potassium.
